(19) Europäisches Patentamt
European Patent Office
Office européen des brevets

(11) **EP 4 503 595 A1**

(12) **EUROPEAN PATENT APPLICATION**
published in accordance with Art. 153(4) EPC

(43) Date of publication:
**05.02.2025 Bulletin 2025/06**

(21) Application number: **23774779.5**

(22) Date of filing: **16.03.2023**

(51) International Patent Classification (IPC):
*H04N 7/14* (2006.01)          *G06F 3/0481* (2022.01)
*G06F 3/0484* (2022.01)        *G06F 3/16* (2006.01)
*G06T 13/20* (2011.01)         *G16H 50/00* (2018.01)
*H04L 67/565* (2022.01)        *H04M 11/00* (2006.01)

(52) Cooperative Patent Classification (CPC):
**G06F 3/0481; G06F 3/0484; G06F 3/16;
G06T 13/20; G16H 50/00; H04L 67/565;
H04M 11/00; H04N 7/14**

(86) International application number:
**PCT/JP2023/010453**

(87) International publication number:
**WO 2023/182183 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.03.2022 JP 2022048112**

(71) Applicant: **Sony Group Corporation**
**Tokyo 108-0075 (JP)**

(72) Inventors:
• **UEHARA, Akira**
  **Tokyo 108-0075 (JP)**
• **SAITO, Mari**
  **Tokyo 108-0075 (JP)**

(74) Representative: **D Young & Co LLP**
**3 Noble Street**
**London EC2V 7BQ (GB)**

(54) **INFORMATION PROCESSING DEVICE AND INFORMATION PROCESSING METHOD**

(57)     An information processing device according to the present disclosure includes: an acquisition unit that acquires first user information related to a first user who is a user on a side to be watched over and second user information related to a second user who is a user on a side of watching over the first user; and a determination unit that determines an abstraction level of presentation information that is information indicating a state of the first user on the basis of the first user information and the second user information.

FIG.6

INFORMATION PROCESSING DEVICE (100)

COMMUNICATION UNIT (110)

CONTROL UNIT (130)
- ACQUISITION UNIT (131)
- PROCESSING UNIT (132)
- DETERMINATION UNIT (133)
- GENERATION UNIT (134)
- TRANSMISSION UNIT (135)

STORAGE UNIT (120)
- DATA STORAGE UNIT (121)
- USER INFORMATION STORAGE UNIT (122)
- ABSTRACTION LEVEL INFORMATION STORAGE UNIT (123)

EP 4 503 595 A1

**Description**

Field

The present disclosure relates to an information processing device and an information processing method.

Background

There is known technology related to provision of services using information. For example, technology for facilitating addition of a newly appearing input or output resource is known (for example, Patent Literature 1).

Citation List

Patent Literature

Patent Literature 1: JP 2006-285308 A

Summary

Technical Problem

However, in the related art, it is not always possible to appropriately determine the abstraction level of information. In the related art, it is possible to add input or output resources; however, the perspective of the abstraction level of information is not considered. For example, in the related art, there is room for improvement such as that an intent of an information providing source, an intent of an information receiver, or the like is not reflected. Therefore, it is desired to appropriately determine the abstraction level of information.
Therefore, the present disclosure proposes an information processing device and an information processing method capable of appropriately determining the abstraction level of information.

Solution to Problem

According to the present disclosure, an information processing device includes an acquisition unit that acquires first user information related to a first user who is a user on a side to be watched over and second user information related to a second user who is a user on a side of watching over the first user; and a determination unit that determines an abstraction level of presentation information that is information indicating a state of the first user on a basis of the first user information and the second user information.

Brief Description of Drawings

FIG. 1 is a diagram illustrating an example of information processing according to an embodiment of the present disclosure.
FIG. 2 is a graph illustrating an example of abstraction level determining processing in an information processing system.
FIG. 3 is a diagram illustrating an example of information regarding a function used for determining the abstraction level.
FIG. 4 is a diagram illustrating an example of information presentation processing in the information processing system.
FIG. 5 is a diagram illustrating a configuration example of an information processing system according to an embodiment of the present disclosure.
FIG. 6 is a diagram illustrating a configuration example of an information processing device according to the embodiment of the disclosure.
FIG. 7 is a diagram illustrating a configuration example of a terminal device according to the embodiment of the disclosure.
FIG. 8 is a flowchart illustrating a processing procedure of the information processing device according to the embodiment of the disclosure.
FIG. 9 is a flowchart illustrating a processing procedure regarding an update of presentation content.

FIG. 10 is a graph illustrating an example of abstraction level determining processing in the information processing system.

FIG. 11 is a diagram illustrating an example of information regarding a function used for determining the abstraction level.

FIG. 12 is a table illustrating examples of sensing targets and presentation modes of information.

FIG. 13 is a diagram illustrating an example of components of the information processing system.

FIG. 14 is a diagram illustrating an example of information presentation.

FIG. 15 is a diagram illustrating an example of information presentation.

FIG. 16 is a hardware configuration diagram illustrating an example of a computer that implements functions of the information processing device. Description of Embodiments

Hereinafter, embodiments of the present disclosure will be described in detail on the basis of the drawings. Note that an information processing device or an information processing method according to the present application is not limited by the embodiments. Note that in each of the following embodiments, the same parts are denoted by the same symbols, and redundant description will be omitted.

The present disclosure will be described in the following order of items.

1. Embodiments

    1-1. Overview of Image Processing According to Embodiment of Present Disclosure

        1-1-1. Exemplary Flow of Information Processing
        1-1-2. Example of Abstraction Level Determining Processing
        1-1-3. Examples of Information Presentation

            1-1-3-1. Protection Level, Attention Degree, and Responsiveness

        1-1-4. Background and Effects

    1-2. Configuration of Information Processing System According to Embodiment
    1-3. Configuration of Information Processing Device According to Embodiment
    1-4. Configuration of Terminal Device According to Embodiment
    1-5. Procedure of Information Processing According to Embodiment

        1-5-1. Procedure of Processing by Information Processing Device
        1-5-2. Procedure of Processing Regarding Update of Presentation Content

    1-6. Examples Regarding Information Processing System

        1-6-1. Processing Example

            1-6-1-1. Plurality of Abstraction Levels
            1-6-1-2. Exemplary Presentation Modes
            1-6-1-3. Examples of Sensing and Data Processing
            1-6-1-4. Sensing and Information Presentation Mode
            1-6-1-5. Examples of Presentation Device and Presentation Content

        1-6-2. System Configuration Example
        1-6-3. Examples of Information Presentation

2. Other Embodiments

    2-1. Other Configuration Examples
    2-2. Others

3. Effects of Present Disclosure
4. Hardware Configuration

[1. Embodiments]

[1-1. Overview of Image Processing According to Embodiment of Present Disclosure]

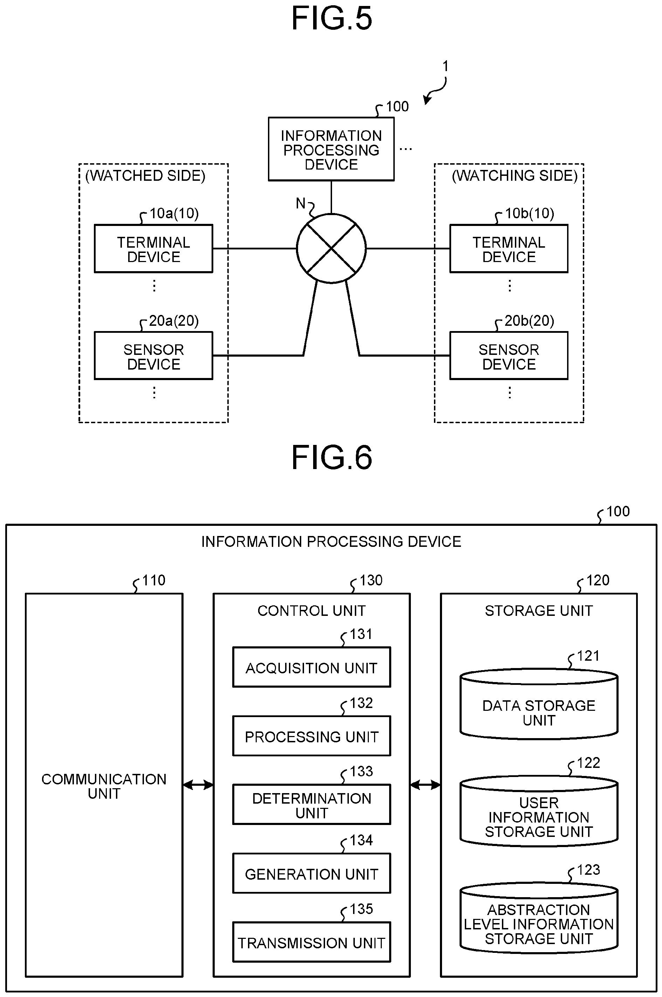

Information processing according to an embodiment of the present disclosure is implemented by an information processing system 1 (see FIG. 5). Although the configuration of the information processing system 1 will be described later, the information processing system 1 executes processing regarding watching over a first user (also referred to as a "watched-side user"), who is on the watched side, by a second user (also referred to as a "watching-side user"), who is on the watching side.

Hereinafter, description will be given on a premise that an elderly person who lives in a nursing home (facility for the elderly) or the like is the first user (watched-side user) and that a family member of the elderly person is the second user (watching-side user). Note that the first user and the second user are merely examples, and the relationship between the first user and the second user is not limited to users having a family relationship. In other words, the first user and the second user may be any users as long as there is a relationship between a side (watched side) from which information is provided to a watching side and a side (watching side) which receives presentation of information of the watched side.

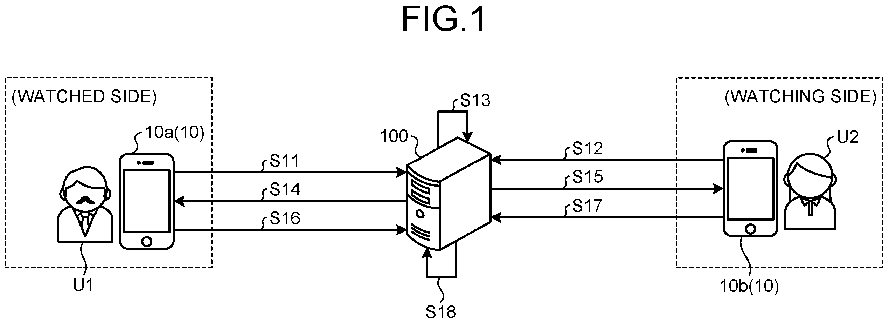

An overview of information processing executed by the information processing system 1 will be described hereinafter with reference to FIGS. 1 to 4.

[1-1-1. Exemplary Flow of Information Processing]

First, a flow of information processing will be described with reference to FIG. 1. FIG. 1 is a diagram illustrating an example of the information processing according to an embodiment of the present disclosure. Hereinafter, a case will be described as an example in which an information processing device 100 determines an abstraction level at a time when presenting, as presentation information, information regarding a user U1 who is the first user.

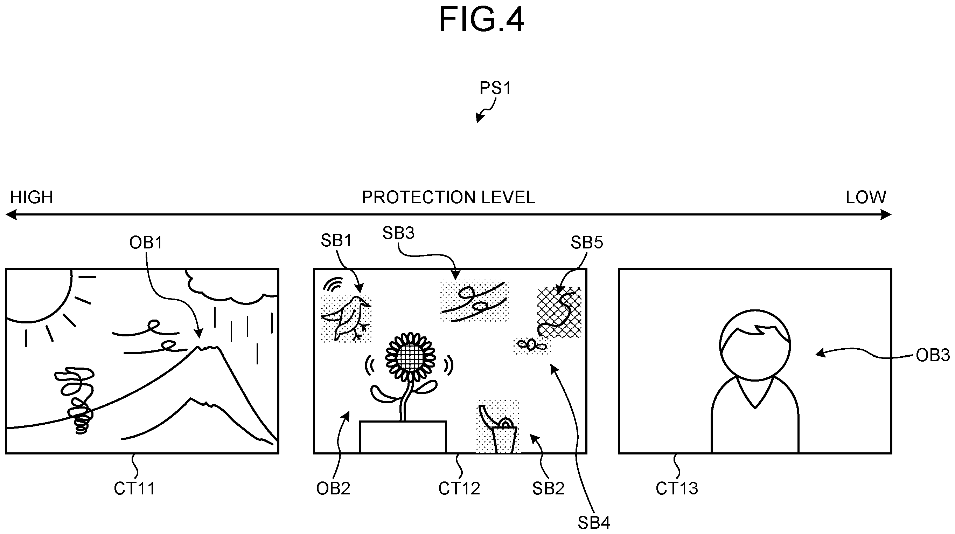

Although details of examples of the presentation information and the like will be described with reference to FIG. 4, the presentation information is based on sensing (detection) regarding the first user (watched-side user) by a sensor device 20a (see FIG. 5). For example, the presentation information is based on a video (also referred to as an "image") capturing the first user. In FIG. 1, the presentation information may be an image based on an image capturing the user U1 who is an elderly person. Note that the above is merely an example, and the presentation information may be any information as long as the information presents the state of the first user. For example, the presentation information may be information based on sound information in which sound around the first user is detected, such as sound information in which sound around the elderly person (activity sound of the elderly person, conversation with a caregiver, etc.) is detected.

The information processing according to the embodiment of the present disclosure is implemented by the information processing system 1 including the information processing device 100, a plurality of sensor devices 20, and a plurality of terminal devices 10. In FIG. 1, a terminal device 10a is a terminal device 10 used by the user U1 who is the first user, and a terminal device 10b is a terminal device 10 used by the user U2 who is the second user who watches over the user U1.

In FIG. 1, a case will be described in which the information processing device 100 determines a mask level common to the first user and the second user as an abstraction level by using first input information input by the first user as first user information and second input information input by the second user as second user information. For example, the mask level that is the abstraction level that has been determined is used to determine how much the information is abstracted when information is presented. Furthermore, in FIG. 1, the information processing device 100 determines an initial value of the abstraction level input by the user U1 who is the first user and an instruction to raise or lower the abstraction level as the first input information. In addition, the information processing device 100 determines an initial value of the abstraction level input by the user U2 who is the second user and an instruction to raise or lower the abstraction level as the second input information.

Note that the abstraction level may be separately determined for the first user and the second user; however, this point will be described later in detail. Furthermore, the first user information used for determining the abstraction level is not limited to the first input information input by the first user and may be information (first sensing information) detected (sensed) by a sensor device 20a. The second user information is not limited to the second input information input by the second user and may be information (second sensing information) detected (sensed) by a sensor device 20b (see FIG. 5).

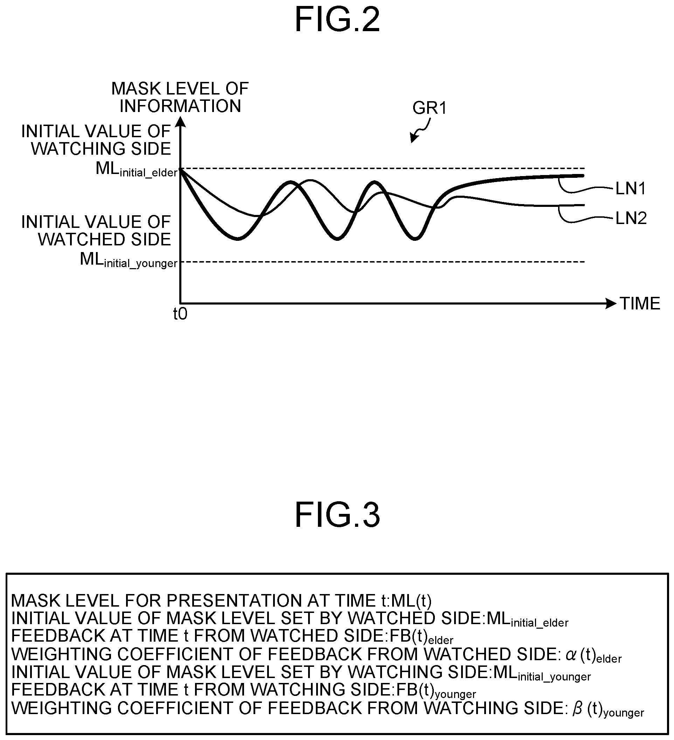

A flow of the processing illustrated in FIG. 1 will be described below. The information processing device 100 acquires an initial value (also referred to as a "first initial value") of the mask level specified by the user U1 on the watched side (step S11). For example, the first initial value is an initial value of the mask level set by the watched side and corresponds to "$ML_{initial\_elder}$" illustrated in FIGS. 2 and 3. The user U1 operates the terminal device 10a to input the first initial value to the terminal device 10a and causes the terminal device 10a to transmit the first initial value. As a result, the terminal device 10a transmits the first initial value input by the user U1 to the information processing device 100, and the information processing device 100 receives the first initial value from the terminal device 10a.

The information processing device 100 also acquires an initial value (also referred to as a "second initial value") of the mask level specified by the user U2 on the watched side (step S12). For example, the second initial value is an initial value of the mask level set on the watching side and corresponds to "$ML_{inital\_younger}$" illustrated in FIGS. 2 and 3. The user U2 operates the terminal device 10b to input the second initial value to the terminal device 10b and causes the terminal device 10b to transmit the second initial value. As a result, the terminal device 10b transmits the second initial value input by the user U2 to the information processing device 100, and the information processing device 100 receives the second initial value from the terminal device 10b. Note that steps S11 and S12 are expediential reference numerals for describing the processing, and the processing of step S12 may be performed before the processing of step S11.

The information processing device 100 determines the abstraction level of the presentation information that is information indicating the state of the first user on the basis of user information (step S13). The information processing device 100 determines the mask level of an image capturing the first user on the basis of the first initial value that is the first user information. For example, the information processing device 100 determines the abstraction level (mask level) on the basis of the first initial value designated by the first user as expressed in the following Equation (1).

$$ML(0) = ML(1) = ML_{initial\_elder} \qquad \cdots (1)$$

ML(0) in Equation (1) corresponds to a mask level at time 0 (corresponding to the origin (t0) in FIG. 2). In addition, ML(1) in Equation (1) corresponds to a mask level at time 1 (time after time 0). In Equation (1), "$ML_{initial\_elder}$" corresponds to a first initial value of the mask level set by the watched side.

Moreover, the information processing device 100 outputs the presentation information corresponding to the abstraction level that has been determined (steps S14 and S15). The information processing device 100 generates presentation information corresponding to the determined mask level and transmits the generated presentation information to the terminal device 10. For example, the information processing device 100 generates content (for example, the content CT11 in FIG. 4) abstracted at the mask level of the first initial value as the presentation information and transmits the generated content to the terminal device 10a and the terminal device 10b. Note that steps S14 and S15 are expediential reference numerals for explaining the processing, and either the processing of step S14 or the processing of step S15 may be performed first or may be performed simultaneously.

Then, the information processing device 100 acquires an instruction to change the mask level by the user U1 as feedback (also referred to as "first feedback") from the user U1 (step S16). For example, the first feedback is feedback (an instruction of raising or lowering the mask level) from the watched side and corresponds to "$FB(t)_{elder}$" illustrated in FIGS. 2 and 3. The user U1 operates the terminal device 10a to input the first feedback to the terminal device 10a and causes the terminal device 10a to transmit the first feedback. As a result, the terminal device 10a transmits the first feedback input by the user U1 to the information processing device 100, and the information processing device 100 receives the first feedback from the terminal device 10a.

Furthermore, the information processing device 100 acquires an instruction to change the mask level by the user U2 as feedback (also referred to as "second feedback") from the user U2 (step S17). For example, the second feedback is feedback (an instruction of raising or lowering the mask level) from the watching side and corresponds to "$FB(t)_{younger}$" illustrated in FIGS. 2 and 3. The user U2 operates the terminal device 10b to input the second feedback to the terminal device 10b and causes the terminal device 10b to transmit the second feedback. As a result, the terminal device 10b transmits the second feedback input by the user U2 to the information processing device 100, and the information processing device 100 receives the second feedback from the terminal device 10b. Note that steps S16 and S17 are expediential reference numerals for describing the processing, and the processing of step S17 may be performed before the processing of step S16.

The information processing device 100 determines the abstraction level of the presentation information on the basis of the first user information and the second user information (step S18). The information processing device 100 determines the mask level of the image capturing the first user on the basis of the first feedback that is the first user information and the second feedback that is the second user information. The information processing device 100 determines (updates) an abstraction level (mask level) on the basis of the first feedback from the first user and the second feedback from the second user, for example, as expressed in the following Equation (2).

$$ML(t+2)$$
$$= ML_{initial\_elder} - \left(\beta(t+1)_{younger} \times FB(t+1)_{younger}\right) + \left(\alpha(t)_{elder} \times FB(t)_{elder}\right) \qquad \cdots (2)$$

ML(t + 2) in Equation (2) corresponds to the mask level at time t + 2. "$ML_{initial\_elder}$" in Equation (2) corresponds to the first initial value of the mask level set by the watched side. "$\beta(t+1)_{younger}$" in Equation (2) corresponds to a weighting coefficient (also referred to as a "second weighting coefficient") of the feedback from the watching side (second feedback) at time t + 1. Note that the second weighting coefficient may be a constant value regardless of time. "FB(t + 1)$_{younger}$" in Equation (2) corresponds to the feedback from the watching side at time t + 1.

In addition, "$\alpha(t)_{elder}$" in Equation (2) corresponds to a weighting coefficient (also referred to as a "first weighting coefficient") of the feedback on the watched side (first feedback) at time t. Note that the first weighting coefficient may be a constant value regardless of time. "FB(t)$_{elder}$" in Equation (2) corresponds to the feedback on the watched side at time t.

Then, the information processing device 100 outputs presentation information corresponding to the updated abstraction level. The information processing device 100 generates presentation information corresponding to the updated mask level and transmits the generated presentation information to the terminal device 10. For example, the information processing device 100 generates content (for example, content CT13 in FIG. 4) abstracted at the mask level calculated by Equation (2) as presentation information and transmits the generated content to the terminal device 10a and the terminal device 10b. For example, in response to the feedback from the first user and the second user, the information processing device 100 repeats determination (update) of the abstraction level in step S18 and output of the presentation information based on the determined (updated) abstraction level.

In this manner, the information processing device 100 can appropriately determine the abstraction level of information by determining the abstraction level of the presentation information on the basis of the first user information and the second user information.

[1-1-2. Example of Abstraction Level Determining Processing]

Hereinafter, an example of abstraction level determining processing will be described with reference to FIGS. 2 and 3. FIG. 2 is a diagram illustrating an example of abstraction level determining processing in the information processing system. FIG. 3 is a diagram illustrating an example of information regarding a function used for determining the abstraction level. Note that description of points similar to the content described in FIG. 1 will be omitted as appropriate. For example, elements illustrated in FIG. 3 are similar to those described in the above-described Equations (1) and (2), and thus description thereof is omitted.

A graph GR1 in FIG. 2 indicates changes along the lapse of time (time series) of the mask level (abstraction level) for the presentation information of the user U1 determined by the information processing device 100. The vertical axis of the graph GR1 indicates the mask level of information, that is, the abstraction level. The horizontal axis of the graph GR1 indicates time.

In the graph GR1, "ML$_{initial\_elder}$" corresponds to the initial value of the mask level set by the watched side. "ML$_{inital\_younger}$" in the graph GR1 corresponds to the initial value of the mask level set by the watching side.

A line LN1 in the graph GR1 indicates a case where the mask level of presentation steadily fluctuates due to input by the watched side and the watching side. In this case, the information processing device 100 determines the mask level at a mask level (abstraction level) desired by the watched side and presents information on the basis of the determined mask level.

In addition, a line LN2 in the graph GR1 indicates a case where the mask level of the presentation fluctuates and attenuates due to input by the watched side and the watching side. In this case, the information processing device 100 determines the mask level in such a manner that the mask level (abstraction level) converges and presents information on the basis of the determined mask level.

For example, in a case where an extent of information that the watched side desires to convey and an extent of information that the watching side desires to view are extremely different, the information processing device 100 sets the initial state in accordance with the request of a user who masks the information extremely. Thereafter, the information processing device 100 adjusts the presentation content on the basis of feedback from users at the time when the mask is gradually removed.

For example, in a case where the watched side does not desire to convey information and the watching side desires to know the information, the information processing device 100 performs the following processing #1-1 to #1-4.

Processing #1-1. Present to both users at high mask level in accordance with watched side.

Processing #1-2. Present with lowered mask level on the basis of input from watching side.
Processing #1-3. Present with raised mask level on the basis of input from watched side.
Processing #1-4. Loop the processing #1-2 and #1-3 to make mask level converge

For example, the information processing device 100 determines the mask level ML(t) of presentation to the watched-side user and the watching-side user at time t by using the above-described Equation (1) or Equation (2). In this manner, the information processing device 100 adjusts the mask level for presentation to the watched-side user and the watching-side user and presents information at the same mask level to both the watched-side user and the watching-side user.

For example, the information processing device 100 multiplies feedback of the watched side and the watching side by weighting, which is set in advance or set in consideration of mutual relationship, personality, the level of symptoms of the watched side, and the like to cause the mask level to fluctuate using addition, subtraction, or an average value. As a result, the information processing device 100 can autonomously and dynamically adjust the mask level for presentation. As described above, the information processing system 1 can autonomously perform matching regarding presentation of the watching side and the watched side by personalization and local optimization.

[1-1-3. Examples of Information Presentation]

Next, an example of information presentation will be described with reference to FIG. 4. FIG. 4 is a diagram illustrating an example of information presentation processing in the information processing system. For example, the information processing device 100 generates at least one piece of content CT11 to CT13 as the presentation information on the basis of the determined abstraction level. The information processing device 100 transmits at least one piece of the generated content CT11 to CT13 to the terminal device 10 as the presentation information. The terminal device 10 displays any one piece of the content CT11 to CT13 received as the presentation information. Note that description of similar points to those described above will be omitted as appropriate.

[1-1-3-1. Protection Level, Attention Degree, and Responsiveness]

The content CT11 to CT13 in FIG. 4 indicates examples of the presentation information. The content CT11 to CT13 in FIG. 4 illustrates examples of changes in the presentation information depending on the protection level.
For example, the content CT11 illustrates an example of presentation information presented in a case where the protection level is high. In the content CT11, an object OB1 imitating a mountain corresponds to the watched-side user (first user). As described above, in the information processing system 1, in the case where the protection level is high, the first user is expressed in a state where the abstraction level is raised.
In addition, the content CT12 illustrates an example of presentation information presented in a case where the protection level is moderate. In the content CT12, an object OB2 imitating a flower corresponds to the watched-side user (first user). As described above, in the information processing system 1, in the case where the protection level is moderate, the first user is expressed in a state where the abstraction level is moderate.
Furthermore, the content CT13 illustrates an example of presentation information presented in a case where the protection level is low. In the content CT13, an object OB3 imitating a person corresponds to the watched-side user (first user). In this manner, in the information processing system 1, in the case where the protection level is low, the first user is expressed in a state where the abstraction level is lowered. Note that the object OB3 in the content CT13 may be a virtual character such as an avatar or an image itself obtained by photographing the first user.
As illustrated in FIG. 4, the protection level corresponds to the abstraction level, and the higher the abstraction level (mask level) is, the higher the protection level is. The information processing device 100 generates presentation information of a higher protection level as the determined abstraction level (mask level) is higher. For example, in a case where the determined abstraction level is greater than or equal to a first threshold value, the information processing device 100 generates the content CT11 with a high protection level as the presentation information. For example, in a case where the determined abstraction level is less than the first threshold value and greater than or equal to the second threshold value, the information processing device 100 generates the content CT12 having a moderate protection level as the presentation information. Note that the second threshold value is smaller than the first threshold value. For example, in a case where the determined abstraction level is less than the second threshold value, the information processing device 100 generates the content CT13 with a low protection level as the presentation information.
As described above, the protection level corresponds to the allowable degree of privacy disclosure and corresponds to the abstraction level of the entire content to be presented. For example, in the case where the protection level is high, the information processing device 100 generates an abstract image as presentation information. Alternatively, in the case where the protection level is moderate, the information processing device 100 generates an illustration as presentation information. Meanwhile, in the case where the protection level is low, the information processing device 100 generates an avatar, a picture of the face only, or the like as presentation information.
As described above, the information processing device 100 determines the abstraction level of the presentation information of the first user depending on the protection level of the presentation information of the first user determined on the basis of the first user information and the second user information. Note that the above is merely an example, and the information processing device 100 may use the protection level for various purposes. For example, the protection level corresponds to an allowable degree of privacy disclosure and corresponds to a theme to be drawn. For example, the information processing device 100 may determine, on the basis of the protection level, the taste of the illustration or a video, the physical transparency, the concreteness or visibility in the mask area, or others.
Furthermore, the information processing device 100 may generate the presentation information depending on the attention degree. The information processing device 100 determines the amount of information of the presentation information of the first user depending on the attention degree of the presentation information of the first user determined on the basis of the first user information and the second user information. For example, the attention degree corresponds to a degree to which the user desires to see and the degree to which the other user desires to convey and corresponds to the type and the number of pieces of object content (also simply referred to as "objects") in the presentation information. For example, the attention degree corresponds to the type of illustration, sound, and haptics.

For example, the information processing device 100 increases the number of objects as the attention degree is higher. The content CT12 in FIG. 4 illustrates an example of the presentation information presented in a case where the attention degree is high. The content CT12 includes a plurality of objects SB1 to SB5 in addition to the object OB2 corresponding to the first user. As described above, in the information processing system 1, in a case where the attention degree is high, the number of objects other than the object corresponding to the first user is raised.

The object SB1 in FIG. 4 is object content imitating a bird poking a flower. The object SB2 in FIG. 4 is object content imitating a watering pot. The object SB3 in FIG. 4 is object content imitating wind. The object SB4 in FIG. 4 is object content imitating a butterfly. The object SB5 in FIG. 4 is object content indicating a traveling locus of the butterfly.

Meanwhile, the information processing device 100 reduces the number of objects as the attention degree is lower. The content CT13 in FIG. 4 illustrates an example of the presentation information presented in a case where the attention degree is low. The content CT13 includes only the object OB3 corresponding to the first user. As described above, in the information processing system 1, in a case where the attention degree is low, the number of objects other than the object corresponding to the first user is reduced. Note that the attention degree may be determined by the information processing device 100 using the first user information or the second user information or may be set in advance.

As described above, the attention degree corresponds to the degree to which the user desires to see and the degree to which the other user desires to convey and corresponds to an object that excites an event. For example, in a case where the protection level is moderate and illustration is used, the information processing device 100 presents information in a mode as the following.

· Bird poking the flower or a bee: conversation with a negative party
· Another flower or a butterfly: conversation with a positive party
· Watering pot: meal or bath
· Weak wind: small amount of activity
· Strong wind: large amount of activity

Furthermore, the information processing device 100 may generate the presentation information depending on the responsiveness. The information processing device 100 determines the resolution of the presentation information of the first user depending on the responsiveness of the presentation information of the first user determined on the basis of the first user information and the second user information. For example, the responsiveness corresponds to a time from occurrence of an event to time when the display content is changed. The information processing device 100 causes the responsiveness to correspond to temporal and spatial resolution in an effect of the content. For example, the information processing device 100 determines the speed, the type, and the like in a motion of a robot, music, illustration, or a shape change of an object on the basis of the responsiveness. For example, the information processing device 100 increases the frame rate of the content as the responsiveness is higher. For example, the information processing device 100 lowers the frame rate of the content as the responsiveness is lower.

As described above, the information processing device 100 associates the time from occurrence of an event to time when display content is changed with the time of an effect of an object on the basis of the responsiveness. For example, in a case where the protection level is moderate and illustration is used and where the responsiveness is high, the information processing device 100 may increase the temporal resolution, increase the frame rate of the effect change, and perform gradation transformation. Furthermore, in a case where the responsiveness is low, the information processing device 100 may switch between display and non-display by lowering the temporal resolution and lowering the frame rate of the effect change. Note that the responsiveness may be determined by the information processing device 100 using the first user information or the second user information or may be set in advance.

As described above, the information processing device 100 maps data and the presentation method, and the information processing device 100 may lower the levels of the protection level, the attention degree, and the responsiveness from high levels on the basis of logs of the user's operations, reactions, or the like. Furthermore, the information processing device 100 may determine the levels of the protection level, the attention degree, and the responsiveness by manual preset by the user. Furthermore, the information processing device 100 may adjust the levels of the protection level, the attention degree, and the responsiveness by, for example, a proposal of an initial value based on a use situation of other users.

For example, in a case where the user enters, approaches, or the like a field of view of a device (also referred to as a "presentation device") that presents information, the information processing device 100 determines that motivation of the user is low and makes the presentation information more ambiguous (raises the abstraction level). Meanwhile, for example, in a case where the user gazes at the presentation device or operates by utterance, a gesture, voice, or manually, or the like, the information processing device 100 may determine that the motivation of the user is high and may make the presentation information more detailed or play back an event in a loop. Furthermore, the information processing system 1 may allow individual registration of content that is created by the user himself or herself with respect to an illustration of the protection level, an object of the attention degree, an effect of the responsiveness, and the like.

Note that the information processing system 1 may continuously change the content depending on a change in the abstraction level. In FIG. 4, the information processing system 1 may continuously change the content C11, CT12, and CT13 depending on a change in the abstraction level. For example, in a case where the abstraction level is raised, the information processing system 1 may cause a continuous change in the order of the content C11, the content CT12, and the content CT13. For example, in a case where the abstraction level is lowered, the information processing system 1 may cause a continuous change in the order of the content C13, the content CT12, and the content CT11.

For example, in a case where the content CT11 continuously changes to the content CT12, the information processing system 1 may zoom in a part of the object OB1 of the mountain. In this case, the information processing system 1 may continuously change the display such that the content CT12 is positioned at a portion where a part of the mountain object OB1 is zoomed in.

Furthermore, for example, in a case where the content CT12 is continuously changed to the content CT13, the information processing system 1 may superimpose the object OB3 of the human on the object OB2 of the flower. In this case, the information processing system 1 may continuously change the display so that the flower object OB2 changes to the human object OB3.

As described above, the information processing system 1 may change the display by organic connection by enlargement or reduction, superimposition, morphing, or the like. The information processing system 1 may change the display in either a continuous or discrete mode.

As described above, the information processing system 1 can convey information reflecting the mutually optimum extent of information discloser by measuring the matching of the content depending on the intent of the watching side and the watched side. In addition, the information processing system 1 can implement an interface having high affinity with the daily life through simple operation and elimination of the reality of the presentation content.

[1-1-4. Background and Effects]

In a conventional system in which an offspring, living away from a parent living in a facility for the elderly, watches over the parent, in a case where the following conditions on the watching side and the watched side are not considered, there is a case where both of the users feel uncomfortable, thereby impairing the usability. For example, conditions of the watching side include the state of the watched side, a relationship with the watched side, the recognition cost of the watching side, and the like. Meanwhile, conditions of the watched side include a privacy protection level desired by the watched side, granularity of information desired to be conveyed, and the like.

For example, in the conventional system, an extent of information that the watching side desires to know and an extent of information that the watched side accepts to be conveyed are not matched for each user, and the content and presentation methods desired by both of the users are not compatible, and thus there is no personalization. In addition, in the conventional system, since no ambient display that achieves both the concreteness of the watching action and the abstractness of privacy protection is dynamically generated, there is no local optimization.

Therefore, the information processing system 1 dynamically changes the mapping between data and the presentation method on the basis of the extent of information that the watching side desires to know and the extent of information that the watched side desires to convey, thereby individually optimizing the presentation to both of the users. For example, the information processing system 1 combines sensors in a facility and scene information including a schedule, performs filtering and dimensionality reduction of conversion, and changes the display content. For example, the information processing system 1 individualizes the nature and the amount of the presentation information on the basis of explicit input or an implicit input from the users.

In the information processing system 1, the amount of exercise, the number of times of opening and closing a door, the number of times of operation of a home appliance, and the like are detected by sensing by a sensor device 20 installed in the environment. Furthermore, in the information processing system 1, the amount of exercise, vitals, utterance content, and the like are detected by sensing by a sensor device 20 of a wearable device.

The information processing system 1 performs data processing. The information processing system 1 performs filtering such as down-sampling, bandpass filtering, outlier removal, and clustering. The information processing system 1 performs conversion such as presence or absence of a change, a difference from others, and dimensionality reduction to an alert to be noted.

The information processing system 1 maps data and the presentation method depending on the watching side and the watched side. The information processing system 1 presents the presentation information based on a protection level indicating an allowable degree of privacy disclosure. The information processing system 1 adjusts, for the watched side, how much information may be disclosed to the watching side.

In addition, the information processing system 1 presents presentation information based on the attention degree indicating the degree that the users desire to view or convey. The information processing system 1 adjusts which piece of information the watching side or the watched side desires to view or convey.

In addition, the information processing system 1 presents the presentation information based on responsiveness indicating a time from occurrence of an event to change of display content. The information processing system 1 adjusts how promptly the watching side or the watched side desires to know and view the information.

As a result, the information processing system 1 can perform communication optimized for mutual motivation on the basis of the degree how much the watching side "desires to know" and the degree how much the watched side "desires to convey". The information processing system 1 can provide comfortable and user-friendly presentation and operation for promoting communication between a resident of a nursing home (watched side) and a family member (watching side). For example, the information processing system 1 makes the allowable level of information discloser from the watched side to match the level of information that the watching side desires to be conveyed and dynamically changes the method of information mapping for an ambient expression. As a result, the information processing system 1 can make the presented information optimized to the motivation of the watching side such as the state of the watched side, a relationship with the watched side, or the recognition cost of the watching side. Furthermore, the information processing system 1 can also make the information optimized to the motivation of the watched side such as a privacy protection level desired by the watched side or the granularity of information desired to be conveyed.

For example, the information processing system 1 constructs a loop of sensing, data processing, and presentation (drawing update). The information processing system 1 performs matching for the protection level, the attention degree, and the responsiveness in presentation on the basis of input from the watched side and the watching side and makes dynamic changes. In the information processing system 1, the input from the user is performed by estimation of a degree based on the behavior of the user or direct operation by the user. The presentation to both of the users in the information processing system 1 may have the same content for both of the users, different content, or content in which pieces of content of both of the users are superimposed on each other.

As described above, in the information processing system 1, the device autonomously and dynamically changes the mapping between the measured or acquired data and the presentation content, and the presentation content is individualized and locally optimized by matching the extent of information that the watching side desires to know and the extent of information that the watched side desires to convey. As a result, the information processing system 1 can implement an interface that is user-friendly to both of a watching-side user and a watched-side user.

Furthermore, the information processing system 1 performs input necessary for individualization of matching between the watched side and the watching side in the presentation in terms of the protection level, the attention degree, and the responsiveness by estimation of a degree based on the behaviors of the users or direct operation from a user. As a result, the information processing system 1 can improve the accuracy of the autonomous and dynamic presentation content by the device.

Furthermore, the information processing system 1 has a configuration in which a unit for sensing the user and the surrounding environment and a unit for presenting information can be recombined for each user and each type of environment. As a result, the information processing system 1 can cope with a variety of characters, ideas, and physical characteristics that are different for each user.

[1-2. Configuration of Information Processing System According to Embodiment]

An information processing system 1 illustrated in FIG. 5 will be described. FIG. 5 is a diagram illustrating a configuration example of an information processing system according to an embodiment. As illustrated in FIG. 5, the information processing system 1 includes an information processing device 100, a terminal device 10a, a terminal device 10b, a sensor device 20a, and a sensor device 20b.

Note that, in a case where the terminal device 10a and the terminal device 10b are described without being particularly distinguished, they are described as "terminal devices 10". Although two terminal devices 10 are illustrated in FIG. 1, three or more terminal devices 10 may be included. In addition, in a case where the sensor device 20a and the sensor device 20b are described without being particularly distinguished, they are described as "sensor devices 20". Although two sensor devices 20 are illustrated in FIG. 1, three or more sensor devices 20 may be included.

The terminal device 10a and the sensor device 20a correspond to the device used on the watched side. For example, the terminal device 10a is a terminal device 10 used by the first user (watched-side user), and the sensor device 20a is a sensor device 20 that performs sensing related to the first user.

The terminal device 10b and the sensor device 20b correspond to devices used on the watching side. For example, the terminal device 10b is a terminal device 10 used by the second user (watching-side user), and the sensor device 20b is a sensor device 20 that performs sensing related to the second user.

The information processing device 100, the terminal device 10, and the sensor device 20 are communicably connected in a wired or wireless manner via a predetermined communication network (network N). Furthermore, the information processing system 1 illustrated in FIG. 5 may include a plurality of information processing devices 100.

The information processing device 100 is a computer processing device that executes processing related to determination of the abstraction level of information. The information processing device 100 determines the abstraction level of the presentation information that is information indicating the state of the first user on the basis of the first user information related to the first user who is the watched-side user and the second user information related to the second user who is on the side of watching over the first user. For example, the information processing device 100 is used to provide various services regarding watching. For example, the information processing device 100 provides a remote monitoring (watching) service of the watched-side user.

The information processing device 100 also has a function of speech recognition. For example, the information processing device 100 has functions of natural language understanding (NLU) or automatic speech recognition (ASR). The information processing device 100 may include software modules such as audio signal processing, speech recognition, utterance semantic analysis, and dialogue control. For example, the information processing device 100 may convert user's utterance into text and estimate the user's utterance content using the utterance converted into text (that is, character information of the utterance). Note that the information processing device 100 may communicate with a speech recognition server having the function of natural language understanding or automatic speech recognition and acquire an utterance converted into text by the speech recognition server or information indicating estimated utterance content from the speech recognition server.

A terminal device 10 is a computer used by a user. The terminal device 10 outputs information regarding watching. The terminal device 10 output information of the watched-side user. The terminal device 10 displays an image (video) related to the watched-side user and outputs sound related to the watched-side user. For example, the terminal device 10 transmits utterance or an image (video) of the user to the information processing device 100 and receives sound or an image (video) of the watched-side user from the information processing device 100.

The terminal device 10 receives input by the user. The terminal device 10 receives voice input by user's utterance or input by a user's operation. The terminal device 10 receives designation of the abstraction level by the user. The terminal device 10 may be any device as long as the processing in the embodiment can be implemented. The terminal device 10 may be any device as long as it has functions of displaying watching information, outputting sound, and the like. For example, the terminal device 10 may be a device such as a lap-top personal computer (PC), a tablet terminal, a desktop PC, a smartphone, a smart speaker, a television, a mobile phone, or a personal digital assistant (PDA).

The terminal device 10 may also have a function of speech recognition such as natural language understanding and automatic speech recognition. For example, the terminal device 10 may convert user's utterance into text and estimate the user's utterance content using the utterance converted into text (that is, character information of the utterance).

A sensor device 20 detects various types of sensor information. The sensor device 20 performs sensing for a user. For example, the sensor device 20 is provided in a space where the user is located. For example, in a case where the first user (watched-side user) is an elderly person living in a facility for the elderly, the sensor device 20a is provided in the facility for the elderly in which the elderly person lives. The sensor device 20 may be worn by the user. For example, the sensor device 20 may be worn on a wrist or the like or worn the neck by the user.

The sensor device 20 includes a sound sensor (microphone) that detects sound. For example, the sensor device 20 detects user's utterance by the sound sensor. The sensor device 20 collects not only the user's utterance but also environmental sound and the like around the sensor device 20. The sensor device 20 includes various sensors without being limited to the sound sensor.

The sensor device 20 has a function as an imaging unit that captures an image. The sensor device 20 has a function of an image sensor and detects image information. The sensor device 20 functions as an image input unit that receives an image as input. For example, the sensor device 20a photographs the first user (watched-side user).

For example, the sensor device 20 may include sensors that detect various types of information such as the temperature, humidity, illuminance, position, acceleration, light, pressure, gyro, or the distance. As described above, the sensor device 20 is not limited to the sound sensor and may include various sensors such as an image sensor (camera) that detects an image, a temperature sensor, a humidity sensor, an illuminance sensor, a position sensor such as a global positioning system (GPS) sensor, an acceleration sensor, an optical sensor, a pressure sensor, a gyro sensor, and a ranging sensor. Furthermore, the sensor device 20 is not limited to the above-described sensors and may include various sensors such as a proximity sensor and a sensor for acquiring biological information such as odor or sent, sweat, heartbeat, pulse, and brain waves.

Moreover, the sensor device 20 may transmit various types of sensor information detected by various sensors to the information processing device 100. Furthermore, the sensor device 20 may include a drive mechanism such as an actuator or a motor with an encoder. The sensor device 20 may transmit sensor information including information detected regarding a drive state or the like of a drive mechanism such as an actuator or a motor with an encoder to the information processing device 100. The sensor device 20 may include software modules such as audio signal processing, speech recognition, utterance semantic analysis, dialogue control, and action output.

Note that the above is an example, and the sensor device 20 may include various sensors without being limited to the above. Incidentally, the sensors that detect the various types of information in the sensor device 20 may be included in a common sensor or may be implemented by different sensors. There may be a plurality of sensor devices 20, and the sensor device 20 has a communication function and transmits collected information (sensing information) to another device such as the information processing device 100.

[1-3. Configuration of Information Processing Device According to Embodiment]

Next, the configuration of the information processing device 100 which is an example of an information processing device that executes the information processing of the embodiment will be described. FIG. 6 is a diagram illustrating a configuration example of the information processing device according to the embodiment of the disclosure.

As illustrated in FIG. 6, the information processing device 100 includes a communication unit 110, a storage unit 120, and a control unit 130. Note that the information processing device 100 may include an input unit (such as a keyboard or a mouse) that receives various operations from the administrator or the like of the information processing device 100 or a display unit (such as a liquid crystal display) for displaying various types of information.

The communication unit 110 is implemented by, for example, a network interface card (NIC) or the like. The communication unit 110 is connected to the network N (see FIG. 5) in a wired or wireless manner and transmits and receives information to and from other information processing devices such as the terminal device 10 and the sensor device 20.

The storage unit 120 is implemented by, for example, a semiconductor memory element such as a random access memory (RAM) or a flash memory or a storage device such as a hard disk or an optical disk. As illustrated in FIG. 6, the storage unit 120 according to the embodiment includes a data storage unit 121, a user information storage unit 122, and an abstraction level information storage unit 123.

The data storage unit 121 according to the embodiment stores various types of information used for processing. The data storage unit 121 stores information to be presented. For example, the data storage unit 121 stores information acquired from the sensor device 20.

The data storage unit 121 stores information indicating the state of the first user detected by the sensor device 20 in association with information indicating the first user and other information. The data storage unit 121 stores information detected by the sensor device 20 in association with information indicating a place where the information has been detected and other information. The data storage unit 121 stores information for identifying each first user (first user ID or the like) in association with user information corresponding to the information.

Note that the data storage unit 121 may store various types of information depending on the purpose without being limited to the above.

The user information storage unit 122 according to the embodiment stores various types of information regarding the user. The user information storage unit 122 stores information indicating the abstraction level designated by each user. The user information storage unit 122 stores information regarding the operation of each user and others. The user information storage unit 122 stores information sensed for each user and others. The user information storage unit 122 stores information for identifying each user (user ID or the like) in association with user information corresponding to the information.

The user information storage unit 122 stores information of the first user. For example, the user information storage unit 122 stores the first user information including the first input information input by the first user. For example, the user information storage unit 122 stores the first input information indicating the abstraction level designated by the first user. The user information storage unit 122 stores the first user information including the first sensing information indicating the state of the first user detected by the sensor device 20.

The user information storage unit 122 stores information of the second user. For example, the user information storage unit 122 stores the second user information including the second input information input by the second user. For example, the user information storage unit 122 stores the second input information indicating the abstraction level designated by the second user. The user information storage unit 122 stores the second user information including the second sensing information indicating the state of the second user detected by the sensor device 20.

Note that the user information storage unit 122 may store various types of information depending on the purpose without being limited to the above. For example, the user information storage unit 122 may store attribute information or the like of each user. The user information storage unit 122 may store information by associating the first user and the second user. The user information storage unit 122 may store information indicating the relationship between the first user and the second user.

The abstraction level information storage unit 123 according to the embodiment stores various types of information regarding the abstraction level. The abstraction level information storage unit 123 stores information indicating the abstraction level of information presentation. The abstraction level information storage unit 123 stores information indicating the abstraction level such as the determined mask level.

Note that the abstraction level information storage unit 123 may store various types of information depending on the purpose without being limited to the above. For example, the abstraction level information storage unit 123 stores a history of the determined abstraction level. For example, the abstraction level information storage unit 123 stores a history of the determined abstraction level in time-series. For example, the abstraction level information storage unit 123 stores the determined abstraction level in association with the first user corresponding to the abstraction level.

The control unit 130 is implemented by, for example, a central processing unit (CPU), a micro processing unit (MPU), or the like executing a program (for example, the information processing program according to the disclosure or the like) stored inside the information processing device 100 using a random access memory (RAM) or the like as a work area. Furthermore, the control unit 130 is implemented by, for example, an integrated circuit such as an application specific integrated circuit (ASIC) or a field programmable gate array (FPGA).

As illustrated in FIG. 6, the control unit 130 includes an acquisition unit 131, a processing unit 132, a determination unit 133, a generation unit 134, and a transmission unit 135 and implements or executes a function or an action of information processing described below. Note that the internal configuration of the control unit 130 is not limited to the configuration illustrated in FIG. 6 and may be another configuration as long as information processing described below is performed. Furthermore, the connection relationship among the processing units included in the control unit 130 is not limited to the connection relationship illustrated in FIG. 6 and may be other connection relationships.

The acquisition unit 131 acquires various types of information. The acquisition unit 131 acquires various types of information from the storage unit 120. The acquisition unit 131 acquires various types of information from an external information processing device. The acquisition unit 131 receives various types of information from an external information processing device via the communication unit 110. The acquisition unit 131 receives information from a terminal device 10. The acquisition unit 131 receives information from a sensor device 20. The acquisition unit 131 acquires various types of information from the terminal device 10 or the sensor device 20. The acquisition unit 131 acquires information collected by the terminal device 10 from the terminal device 10. The acquisition unit 131 acquires information detected by the sensor device 20 from the sensor device 20.

The acquisition unit 131 acquires various types of information regarding a user. The acquisition unit 131 acquires information regarding the first user to be presented to the second user. The acquisition unit 131 acquires information indicating the state of a user detected by the sensor device 20. The acquisition unit 131 acquires information regarding the state of the first user who is the watched-side user. The acquisition unit 131 acquires information indicating a sensing result of the first user by a sensor. The acquisition unit 131 acquires information regarding the activity of the first user. The acquisition unit 131 acquires information regarding the amount of activity of the first user.

The acquisition unit 131 acquires the first user information regarding the first user who is the watched-side user. The acquisition unit 131 acquires the first user information including the first input information input by the first user. The acquisition unit 131 acquires the first input information indicating the abstraction level specified by the first user. The acquisition unit 131 acquires the first input information indicating an instruction to lower the abstraction level by the first user. The acquisition unit 131 acquires the first input information indicating an instruction to raise the abstraction level by the first user. The acquisition unit 131 acquires the first user information including the first sensing information indicating the state of the first user detected by the sensor device 20.

The acquisition unit 131 acquires the second user information regarding the second user who is a user on the side of watching over the first user. The acquisition unit 131 acquires the second user information including the second input information input by the second user. The acquisition unit 131 acquires the second input information indicating the abstraction level specified by the second user. The acquisition unit 131 acquires the second input information indicating an instruction to lower the abstraction level by the second user. The acquisition unit 131 acquires the second input information indicating an instruction to raise the abstraction level by the second user. The acquisition unit 131 acquires the second user information including the second sensing information indicating the state of the second user detected by the sensor device 20.

The processing unit 132 executes various types of processing. The processing unit 132 executes processing using the information acquired by the acquisition unit 131. The processing unit 132 executes image processing. The processing unit 132 executes processing related to speech recognition. The processing unit 132 executes speech recognition processing using information stored in the storage unit 120. The processing unit 132 converts user's utterance into text by converting the user's utterance into character information. The processing unit 132 can be implemented by using existing utterance semantic analysis technology.

Furthermore, the processing unit 132 analyzes the content of the user's utterance. The processing unit 132 estimates the content of the user's utterance by analyzing the user's utterance using various types of conventional technology as appropriate. For example, the processing unit 132 analyzes the content of the user's utterance by the functions of natural language understanding (NLU) and automatic speech recognition (ASR). The processing unit 132 estimates (specifies) the content of the user's utterance by semantic analysis using character information corresponding to the user's utterance. For example, the processing unit 132 estimates the content of the user's utterance corresponding to the character information by analyzing character information using various types of conventional technology as appropriate, such as syntax analysis.

The processing unit 132 executes processing related to data holding. The processing unit 132 accumulates information transmitted from each terminal device 10 or sensor device 20. The processing unit 132 accumulates information such as a recognition result of sensing information such as an image or voice transmitted from each terminal device 10 or sensor device 20. The processing unit 132 stores, in the storage unit 120, information such as a recognition result of sensing information such as an image or voice transmitted from each terminal device 10 or sensor device 20. The processing unit 132 executes keyword extraction processing. The processing unit 132 extracts a keyword on the basis of the result of speech recognition.

The determination unit 133 determines various types of information. The determination unit 133 determines the abstraction level of information. The determination unit 133 executes determination processing using the information acquired by the acquisition unit 131.

The determination unit 133 determines the abstraction level of the presentation information that is information indicating the state of the first user on the basis of the first user information and the second user information. The determination unit 133 determines the abstraction level of the presentation information of the first user on the basis of the first input information. The determination unit 133 determines the abstraction level of the presentation information of the first user on the basis of the abstraction level indicated by the first input information. The determination unit 133 lowers the abstraction level of the presentation information of the first user in accordance with an instruction to lower the abstraction level by the first user. The determination unit 133 raises the abstraction level of the presentation information of the first user in accordance with an instruction to raise the abstraction level by the first user. The determination unit 133 determines the abstraction level of the presentation information of the first user on the basis of the first sensing information.

The determination unit 133 determines the abstraction level of the presentation information of the second user on the basis of the second input information. The determination unit 133 determines the abstraction level of the presentation information of the second user on the basis of the abstraction level indicated by the second input information. The determination unit 133 lowers the abstraction level of the presentation information of the second user in accordance with an instruction to lower the abstraction level by the second user. The determination unit 133 raises the abstraction level of the presentation information of the second user in accordance with an instruction to raise the abstraction level by the second user. The determination unit 133 determines the abstraction level of the presentation information of the second user on the basis of the second sensing information.

The determination unit 133 determines the abstraction level at the time of presenting the presentation information of the first user to the second user on the basis of the first user information and the second user information. The determination unit 133 determines the abstraction level at the time of presenting the presentation information of the first user to the first user on the basis of the first user information and the second user information.

The determination unit 133 determines the abstraction level of the presentation information of the first user depending on the protection level of the presentation information of the first user determined on the basis of the first user information and the second user information. The determination unit 133 determines the amount of information of the presentation information of the first user depending on the attention degree of the presentation information of the first user determined on the basis of the first user information and the second user information. The determination unit 133 determines the resolution of the presentation information of the first user depending on the responsiveness of the presentation information of the first user determined on the basis of the first user information and the second user information.

The generation unit 134 generates various types of information. The generation unit 134 generates various types of information on the basis of information from an external information processing device or information stored in the storage unit 120. The generation unit 134 generates various types of information on the basis of information from other information processing devices such as terminal devices 10 and sensor devices 20. The generation unit 134 generates various types of information on the basis of information stored in the data storage unit 121, the user information storage unit 122, or the abstraction level information storage unit 123. The generation unit 134 generates various types of information to be output to the terminal devices 10 on the basis of the information generated by the processing by the processing unit 132.

The generation unit 134 executes various types of processing related to information to be provided to the terminal devices 10. The generation unit 134 generates content to be provided to the terminal devices 10. The generation unit 134 generates content corresponding to the abstraction level determined by the determination unit 133. The generation unit 134 generates the content CT11. The generation unit 134 generates the content CT12. The generation unit 134 generates the content CT13.

In addition, the generation unit 134 generates the content to be displayed on the terminal devices 10. For example, the generation unit 134 may generate a screen (content) to be provided to the terminal devices 10 by using various types of technology such as Java (registered trademark) as appropriate. Note that the generation unit 134 may generate a screen (content) to be provided to the terminal devices 10 on the basis of a format such as CSS, JavaScript (registered trademark), or HTML. Furthermore, for example, the generation unit 134 may generate a screen (content) in various formats such as joint photographic experts group (JPEG), graphics interchange format (GIF), or portable network graphics (PNG).

The transmission unit 135 functions as an output unit that executes output processing. The transmission unit 135 transmits information to the terminal devices 10. The transmission unit 135 transmits information indicating a processing result by the processing unit 132 to the terminal devices 10. The transmission unit 135 transmits the information of the abstraction level determined by the determination unit 133 to the terminal device 10. The transmission unit 135 transmits information generated by the generation unit 134 to the terminal devices 10. The transmission unit 135 transmits the content generated by the generation unit 134 to the terminal devices 10.

The transmission unit 135 outputs presentation information corresponding to the abstraction level determined by the determination unit 133. The transmission unit 135 transmits the content CT11. The transmission unit 135 transmits the content CT12. The transmission unit 135 transmits the content CT13. The transmission unit 135 transmits the presentation information corresponding to the abstraction level determined by the determination unit 133 to the terminal device 10 used by the second user. The transmission unit 135 transmits the presentation information corresponding to the abstraction level determined by the determination unit 133 to the terminal device 10 used by the first user.

[1-4. Configuration of Terminal Device According to Embodiment]

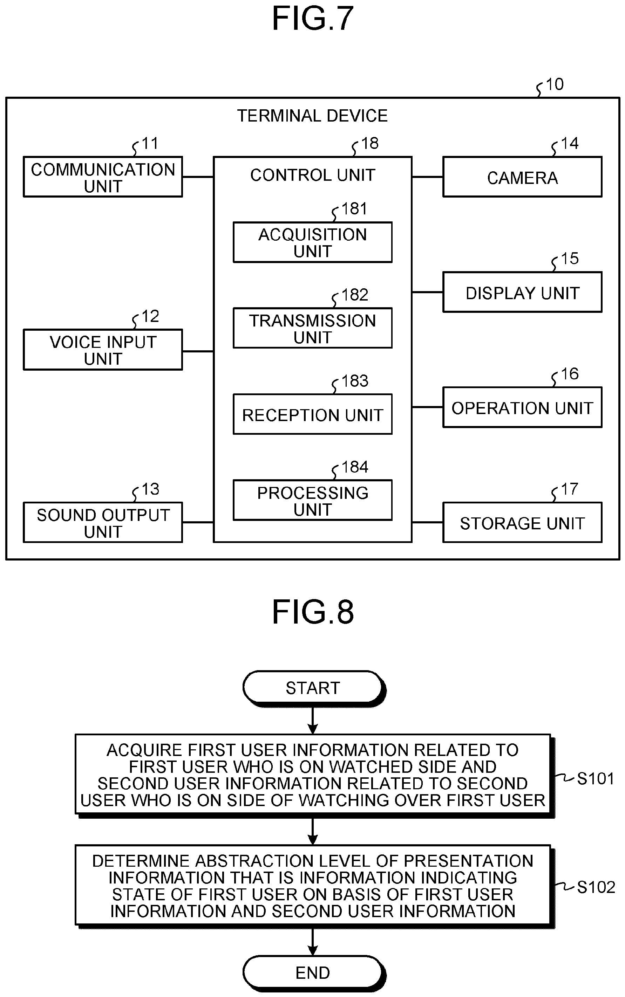

Next, a configuration of a terminal device 10 which is an example of an output device that executes output processing according to the embodiment will be described. FIG. 7 is a diagram illustrating a configuration example of a terminal device according to the embodiment of the disclosure.

As illustrated in FIG. 7, the terminal device 10 includes a communication unit 11, a voice input unit 12, a sound output unit 13, a camera 14, a display unit 15, an operation unit 16, a storage unit 17, and a control unit 18.

The communication unit 11 is implemented by, for example, an NIC, a communication circuit, or the like. The communication unit 11 is connected with a predetermined communication network in a wired or wireless manner and transmits and receives information to and from an external information processing device. For example, the communication unit 11 is connected with a predetermined communication network in a wired or wireless manner and transmits and receives information to and from the information processing device 100.

The voice input unit 12 functions as an input unit that receives an operation by user's voice (utterance). The voice input unit 12 is, for example, a microphone or the like, and detects the voice. For example, the voice input unit 12 detects user's utterance. The voice input unit 12 receives user's utterance as an operation by the user. The voice input unit 12 receives designation of the abstraction level of the presentation information from the user using the terminal device 10. Note that the voice input unit 12 may have any configuration as long as it can detect user's utterance information necessary for processing.

The sound output unit 13 is implemented by a speaker that outputs sound and is an output device for outputting various types of information as sound. The sound output unit 13 outputs, by sound, the content provided from the information processing device 100. For example, the sound output unit 13 outputs sound corresponding to the information displayed on the display unit 15. The terminal device 10 inputs and outputs sound using the voice input unit 12 and the sound output unit 13. For example, the sound output unit 13 outputs, by sound, the presentation information corresponding to the determined abstraction level.

The camera 14 includes an image sensor (image sensor) that detects an image. The camera 14 captures an image. For example, in a case where the terminal device 10 is a laptop computer, the camera 14 may be built in the terminal device 10 and disposed above the display unit 15. Alternatively, for example, in a case of a smartphone, the camera 14 may be a front camera built in the terminal device 10.

The display unit 15 is a display screen such as a tablet terminal implemented by, for example, a liquid crystal display, an organic electro-luminescence (EL) display, or the like and is a display device for displaying various types of information.

The display unit 15 functions as an output unit that executes output processing. The display unit 15 displays various types of information regarding watching. The display unit 15 displays various types of information regarding the first user. The display unit 15 displays content. The display unit 15 displays various types of information received from the information processing device 100.

The display unit 15 outputs presentation information corresponding to the abstraction level determined by the information processing device 100. The display unit 15 displays the presentation information corresponding to the abstraction level determined by the information processing device 100.

The display unit 15 displays the content CT11. The display unit 15 displays the content CT12. The display unit 15 displays the content CT13.

The operation unit 16 functions as an input unit that receives various user operations. The operation unit 16 receives an operation with respect to the information displayed by the display unit 15 from the user using the terminal device 10. The operation unit 16 receives designation of the abstraction level of the presentation information from the user using the terminal device 10.

In the example of FIG. 7, the operation unit 16 is a keyboard, a mouse, or the like. Furthermore, the operation unit 16 may have a touch panel capable of implementing functions equivalent to those of a keyboard and a mouse. In this case, the operation unit 16 receives various operations from the user via the display screen by the function of the touch panel implemented by various sensors. For example, the operation unit 16 receives various operations from the user via the display unit 15.

For example, the operation unit 16 receives an operation such as a designation operation by the user via the display unit 15 of the terminal device 10. Note that, as a method of detecting the user's operation by the operation unit 16, an electrostatic capacitance method is mainly adopted in a tablet terminal; however, any method may be adopted as long as the user's operation can be detected and the function of the touch panel can be implemented, such as a resistive film method, a surface acoustic wave method, an infrared method, or an electromagnetic induction method, which are other detection methods.

The above keyboard, mouse, touch panel, and the like are merely examples, and the terminal device 10 may have a configuration of receiving (detecting) various types of information as an input without being limited to the above. For example, the terminal device 10 may have a line-of-sight sensor that detects the line of sight of the user. The line-of-sight sensor detects the line-of-sight direction of the user using the eye tracking technology on the basis of detection results of the camera 14, an optical sensor, a motion sensor (all not illustrated), and the like mounted on the terminal device 10, for example. The line-of-sight sensor determines a gaze region at which the user is gazing on the screen on the basis of the detected line-of-sight direction. The line-of-sight sensor may transmit the line-of-sight information including the determined gaze region to the information processing device 100. For example, the terminal device 10 may include a motion sensor that detects a gesture or the like of the user. The terminal device 10 may receive an operation by a gesture of the user by the motion sensor.

The storage unit 17 is implemented by, for example, a semiconductor memory element such as a random access memory (RAM) or a flash memory or a storage device such as a hard disk or an optical disk. The storage unit 17 stores, for example, various types of information received from the information processing device 100. The storage unit 17 stores, for example, information regarding an application (for example, a watching application or the like) installed in the terminal device 10 such as a program.

The storage unit 17 stores information received from the information processing device 100. The storage unit 17 stores presentation information to be presented to the user. The storage unit 17 stores content to be output. For example, the storage unit 17 stores user information. In this case, the storage unit 17 may store an utterance history (history of speech recognition results) or an action history of the user.

The control unit 18 is implemented by, for example, a CPU, an MPU, or the like executing various programs stored in a storage device such as the storage unit 17 inside the terminal device 10 using the RAM as a work area. For example, the various programs include a program of an application (for example, a watching application) that performs information processing. Note that the control unit 18 is implemented by an integrated circuit such as an ASIC or an FPGA.

As illustrated in FIG. 7, the control unit 18 includes an acquisition unit 181, a transmission unit 182, a reception unit 183, and a processing unit 184 and implements or executes a function or an action of information processing described below. Note that the internal configuration of the control unit 18 is not limited to the configuration illustrated in FIG. 7 and may be another configuration as long as information processing described below is performed. Furthermore, the connection relationship among the processing units included in the control unit 18 is not limited to the connection relationship illustrated in FIG. 7 and may be other connection relationships.

The acquisition unit 181 acquires various types of information. For example, the acquisition unit 181 acquires various types of information from an external information processing device. For example, the acquisition unit 181 stores the acquired various types of information in the storage unit 17. The acquisition unit 181 acquires user's operation information received by the operation unit 16. The acquisition unit 181 acquires presentation information to be presented to the user.

The acquisition unit 181 acquires utterance information of the user. The acquisition unit 181 acquires the utterance information of the user detected by the voice input unit 12.

The transmission unit 182 transmits information to the information processing device 100 via the communication unit 11. The transmission unit 182 transmits information regarding the user to the information processing device 100. The transmission unit 182 transmits information input by user's utterance, operation, or the like. The transmission unit 182 transmits information indicating an operation with respect to the presentation information to the information processing device 100.

The reception unit 183 receives information from the information processing device 100 via the communication unit 11. The reception unit 183 receives information provided by the information processing device 100. The reception unit 183 receives content from the information processing device 100. The reception unit 183 receives the presentation information of the abstraction level determined by the information processing device 100.

The processing unit 184 executes various types of processing. The processing unit 184 executes processing using the information provided from the information processing device 100. The processing unit 184 displays various types of information via the display unit 15. For example, the processing unit 184 functions as a display control unit that controls display of the display unit 15. The processing unit 184 outputs various types of information by sound via the sound output unit 13. For example, the processing unit 184 functions as a sound output control unit that controls sound output of the sound output unit 13.

The processing unit 184 changes the display mode in accordance with the user's operation received by the voice input unit 12 or the operation unit 16. The processing unit 184 outputs the information acquired by the acquisition unit 181. The processing unit 184 outputs the information received by the reception unit 183. The processing unit 184 outputs content provided from the information processing device 100. The processing unit 184 outputs the content received by the reception unit 183 via the sound output unit 13 or the display unit 15. The processing unit 184 displays the content via the display unit 15. The processing unit 184 outputs the content by sound via the sound output unit 13.

The processing unit 184 transmits various types of information to an external information processing device via the communication unit 11. The processing unit 184 transmits various types of information to the information processing device 100. The processing unit 184 transmits various types of information stored in the storage unit 17 to an external information processing device. The processing unit 184 transmits various types of information acquired by the acquisition unit 181 to the information processing device 100. The processing unit 184 transmits sensor information acquired by the acquisition unit 181 to the information processing device 100. The processing unit 184 transmits operation information of the user received by the operation unit 16 to the information processing device 100. The processing unit 184 transmits information such as utterance or images of the user using the terminal device 10 to the information processing device 100.

Note that each piece of processing by the control unit 18 described above may be implemented by, for example, JavaScript (registered trademark) or the like. Furthermore, in a case where the processing such as information processing by the control unit 18 described above is performed by a predetermined application, each of the units of the control unit 18 may be implemented by, for example, the predetermined application. For example, the processing such as information processing by the control unit 18 may be implemented by control information received from an external information processing device. For example, in a case where the above-described display processing is performed by the predetermined application (such as a watching application), the control unit 18 may include, for example, an application control unit that controls the predetermined application or a dedicated application.

[1-5. Procedure of Information Processing According to Embodiment]

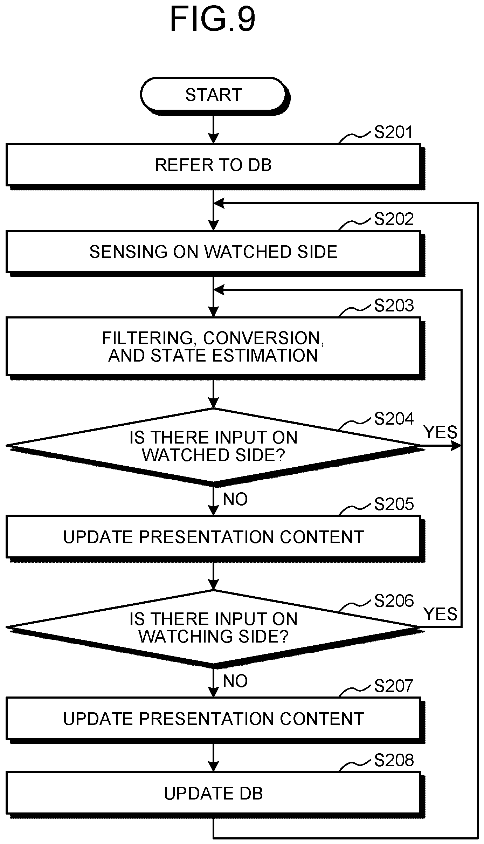

Next, procedures of various types of information processing according to the embodiment will be described by referring to FIGS. 8 and 9.

[1-5-1. Procedure of Processing by Information Processing Device]

First, the flow of processing by the information processing device will be described with reference to FIG. 8. FIG. 8 is a flowchart illustrating a processing procedure of the information processing device according to the embodiment of the disclosure. Specifically, FIG. 8 is a flowchart illustrating a procedure of information processing by the information processing device 100 which is an example of the information processing device.

As illustrated in FIG. 8, the information processing device 100 acquires the first user information related to the first user who is on the side of being watched over and the second user information related to the second user who is on the side of watching over the first user (step S101). The information processing device 100 determines the abstraction level of the presentation information that is information indicating the state of the first user on the basis of the first user information and the second user information (step S102).

[1-5-2. Procedure of Processing Regarding Update of Presentation Content]

Next, processing related to an update of presentation content will be described with reference to FIG. 9. FIG. 9 is a flowchart illustrating a processing procedure related to an update of the presentation content. Note that, in the following, a case where the information processing system 1 performs processing will be described as an example; however, the processing illustrated in FIG. 9 may be performed by any device such as the information processing device 100, the terminal device 10, or the sensor device 20 depending on a device configuration included in the information processing system 1.

In FIG. 9, the information processing system 1 refers to a DB (step S201). For example, the information processing device 100 refers to information stored in the storage unit 120. The information processing system 1 performs sensing on the watched side (step S202). For example, a sensor device 20 performs sensing. The information processing system 1 performs various types of processing such as filtering, conversion, and state estimation (step S203). For example, the information processing device 100 performs various types of processing such as filtering, conversion, and

17

state estimation.

If there is input on the watched side (step S204: Yes), the information processing system 1 returns to step S203 and performs processing. If there is no input on the watched side (step S204: No), the information processing system 1 updates the presentation content (step S205). For example, the information processing device 100 determines the abstraction level of the presentation information, and the terminal device 10 updates the information to be displayed in the presentation information based on the abstraction level determined by the information processing device 100.

If there is input on the watching side (step S206: Yes), the information processing system 1 returns to step S203 and performs processing. If there is no input on the watching side (step S206: No), the information processing system 1 updates the presentation content (step S207). For example, the information processing device 100 determines the abstraction level of the presentation information, and the terminal device 10 updates the information to be displayed in the presentation information based on the abstraction level determined by the information processing device 100.

Then, the information processing system 1 updates the DB (step S208) and returns to step S202 to perform processing. For example, the information processing device 100 updates the information stored in the storage unit 120.

[1-6. Examples Regarding Information Processing System]

Hereinafter, processing examples and the like performed by the information processing system 1 will be described. Note that description of similar points to those described above will be omitted as appropriate.

[1-6-1. Processing Example]

First, a processing example performed by the information processing system 1 will be described.

[1-6-1-1. Plurality of Abstraction Levels]

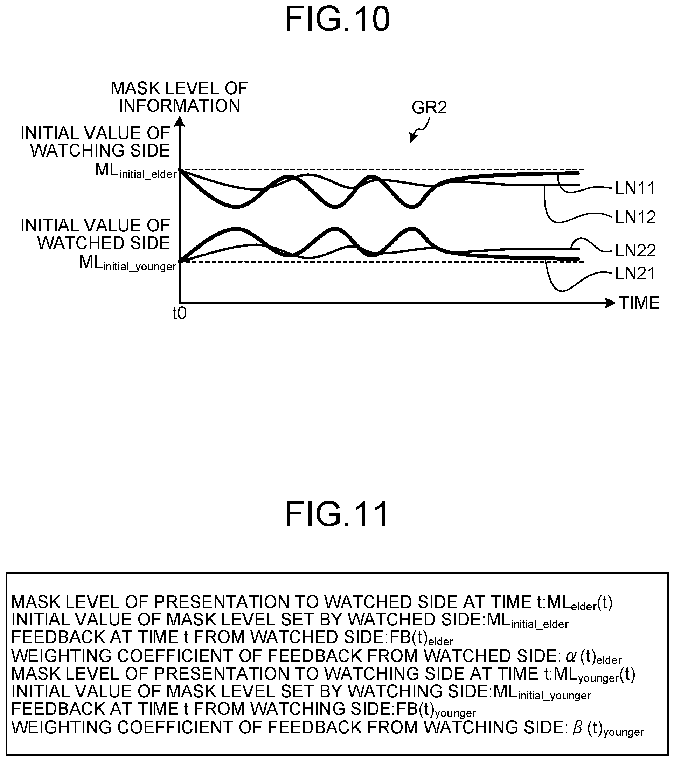

In the above-described example, a case where a mask level common to the watched-side user (first user) and the watching-side user (second user) is used has been described as an example. However, the information processing device 100 may separately determine the mask level (abstraction level) for the first user and the second user. This point will be described with reference to FIGS. 10 and 11.

FIG. 10 is a diagram illustrating an example of abstraction level determining processing in the information processing system. FIG. 11 is a diagram illustrating an example of information regarding a function used for determining the abstraction level. Note that a mask level (abstraction level) for the watched-side user (first user) is also referred to as a first mask level (first abstraction level), and a mask level (abstraction level) for the watching-side user (second user) is also referred to as a second mask level (second abstraction level).

The information processing device 100 determines the first mask level (first abstraction level) of the watched-side user (first user). For example, the information processing device 100 determines a mask level $ML_{elder}(t)$, which is the first mask level for presentation to the watched side at time t, by using the following Equation (3) or Equation (4). For example, the information processing device 100 determines the first mask level (first abstraction level) on the basis of the first initial value designated by the first user as expressed in the following Equation (3).

$$ML_{elder}(0) = ML_{elder}(1) = ML_{initial\_elder} \qquad \cdots (3)$$

$ML_{elder}(0)$ in Equation (3) corresponds to the first mask level at time 0 (corresponding to the origin (t0) in FIG. 2). Furthermore, $ML_{elder}(3)$ in Equation (3) corresponds to the first mask level at time 1 (time after time 0). "$ML_{initial\_elder}$" in Equation (3) corresponds to the first initial value of the first mask level set by the watched side.

For example, the information processing device 100 determines (updates) the first mask level on the basis of the first feedback from the first user and the second feedback from the second user as expressed in the following Equation (4).

$$ML_{elder}(t+2)$$
$$= ML_{initial\_elder} - \left(\beta(t+1)_{younger} \times FB(t+1)_{younger}\right) + \left(\alpha(t)_{elder} \times FB(t)_{elder}\right) \qquad \cdots (4)$$

$ML_{elder}(t+2)$ in Equation (4) corresponds to the first mask level at time t + 2. In addition, "$ML_{initial\_elder}$" in Equation (4) corresponds to the first initial value of the first mask level set by the watched side. Note that the other elements in Equation (4) are similar to those in Equation (2), and thus detailed description thereof is omitted.

The information processing device 100 determines the second mask level (second abstraction level) of the watching-side user (second user). For example, the information processing device 100 determines a mask level $ML_{younger}$

(t), which is the second mask level for presentation to the watching side at time t, by using the following Equation (5) or Equation (6). For example, the information processing device 100 determines the second mask level (second abstraction level) on the basis of the second initial value designated by the second user as expressed in the following Equation (5).

$$\mathrm{ML}_{\mathrm{younger}}\ (0) = \mathrm{ML}_{\mathrm{younger}}\ (1) = \mathrm{ML}_{\mathrm{initial\ \_younger}} \qquad \cdots (5)$$

$\mathrm{ML}_{\mathrm{younger}}(0)$ in Equation (5) corresponds to the second mask level at time 0 (corresponding to the origin (t0) in FIG. 2) . Furthermore, $\mathrm{ML}_{\mathrm{younger}}$ (5) in Equation (5) corresponds to the second mask level at time 1 (time after time 0). "$\mathrm{ML}_{\mathrm{initial\_younger}}$" in Equation (5) corresponds to the second initial value of the second mask level set by the watched side.
For example, the information processing device 100 determines (updates) the second mask level on the basis of the first feedback from the first user and the second feedback from the second user as expressed in the following Equation (6).

$$\mathrm{ML}_{\mathrm{younger}}\ (t+2)$$
$$= \mathrm{ML}_{\mathrm{initial\ \_younger}} - \left(\beta(t+1)_{\mathrm{younger}} \times \mathrm{FB}(t+1)_{\mathrm{younger}}\right) + \left(\alpha(t)_{\mathrm{elder}} \times \mathrm{FB}(t)_{\mathrm{elder}}\right) \qquad \cdots (6)$$

$\mathrm{ML}_{\mathrm{younger}}$ (t + 2) in Equation (6) corresponds to the second mask level at time t + 2. In addition, "$\mathrm{ML}_{\mathrm{initial\_younger}}$" in Equation (6) corresponds to the second initial value of the second mask level set by the watched side. Note that the other elements in Equation (6) are similar to those in Equation (2), and thus detailed description thereof is omitted.
A graph GR2 in FIG. 10 indicates changes along the lapse of time (time series) of the mask level (abstraction level) for the presentation information of the user U1 determined by the information processing device 100. The vertical axis of the graph GR2 indicates the mask level of information, that is, the abstraction level. The horizontal axis of the graph GR2 indicates time.
In the graph GR2, "$\mathrm{ML}_{\mathrm{initial\_elder}}$" corresponds to the initial value of the mask level set by the watched side. "$\mathrm{ML}_{\mathrm{inital\_younger}}$" in the graph GR2 corresponds to the initial value of the mask level set by the watching side.
A line LN11 and a line LN21 in the graph GR2 indicate a case where the mask level of presentation steadily fluctuates due to input by the watched side and the watching side. The line LN11 corresponds to the first mask level, and a line LN12 corresponds to the second mask level. In this case, the information processing device 100 determines the first mask level at a mask level (abstraction level) desired by the watched side and presents information to the first user on the basis of the determined first mask level. Furthermore, the information processing device 100 determines the second mask level at a mask level (abstraction level) desired by the watching side and presents information to the second user on the basis of the determined second mask level.
In addition, the line LN12 and a line LN22 in the graph GR2 indicates a case where the mask level of the presentation fluctuates and attenuates due to input by the watched side and the watching side. The line LN21 corresponds to the first mask level, and the line LN22 corresponds to the second mask level. In this case, the information processing device 100 determines the first mask level in such a manner that the mask level (abstraction level) converges and presents information to the first user on the basis of the determined first mask level. Furthermore, the information processing device 100 determines the second mask level in such a manner that the mask level (abstraction level) converges and presents information to the second user on the basis of the determined second mask level.
For example, in a case where an extent of information that the watched side desires to convey and an extent of information that the watching side desires to view are extremely different, the information processing device 100 sets the initial state in accordance with the request of a user who masks the information extremely. Thereafter, the information processing device 100 adjusts the presentation content on the basis of feedback from users at the time when the mask is gradually removed.
For example, in a case where the watched side desires to convey information and the watching side does not desire to know the information, the information processing device 100 performs the following processing #2-1 to #2-4.
Processing #2-1. Present at mask levels requested by the watched side and the watching side

Processing #2-2. On the basis of the input by the watched side, the mask level of the watching side is lowered and perform presentation
Processing #2-3. On the basis of the input by the watching side, the mask level of the watching side is lowered and perform presentation
Processing #2-4. Loop the processing #2-2 and #2-3 to make mask level converge

For example, the information processing device 100 determines the mask level $\mathrm{ML}_{\mathrm{elder}}(t)$ of presentation to the watched-side user at time t by using the above-described Equation (3) or Equation (4). Furthermore, the information processing device 100 determines the mask level $ML_{younger}(t)$ of presentation to the watched-side user at time t by using the above-described Equation (5) or Equation (6). In this manner, the information processing device 100 adjusts the mask levels for presentation to the watched-side user and the watching-side user and presents information at separate mask levels for the watched-side user and the watching-side user. As described above, the information processing system 1 can autonomously perform matching regarding presentation of the watching side and the watched side by personalization and local optimization.

[1-6-1-2. Exemplary Presentation Modes]

The above-described presentation mode of information is merely an example, and the information processing system 1 may present information in various presentation modes. An example of this point will be described below. Hereinafter, description will be given on an example of presentation method in a situation where watching-side users who request different mask levels are present in the same environment.

In a case where watching-side users each browse information of the watched-side user, the information processing system 1 may perform processing as follows. For example, the information processing system 1 performs presentation by setting the mask level of information to the highest mask level set in advance on the basis of input by the watching user. Furthermore, the information processing system 1 adjusts the mask level on the basis of an operation by the watching-side user or a recognition result of the watching-side user by a device and perform presentation.

For example, in a case where a genetic offspring of the watched-side user and a spouse of the offspring live together, the information processing system 1 may perform processing as follows. For example, in a case where the genetic offspring who desires to know details requests (designates) a low mask level and the spouse of the offspring who does not need to know the details requests a high mask level, the information processing system 1 presents information by a terminal device 10 at the low mask level to be in line with the spouse of the offspring. Furthermore, when the genetic offspring accesses the watched-side user, the information processing system 1 may specify the user by an operation or identification such as biometric authentication using a fingerprint, utterance, or iris or facial feature amount analysis and perform presentation by temporarily adjusting to the mask level registered in advance.

In a case where watching-side users each browse information of the watched-side user simultaneously, the information processing system 1 may perform processing as follows. For example, the information processing system 1 performs presentation by setting the mask level of information to the highest mask level set in advance on the basis of input by the watching user. Note that in a case where the configuration of the presentation device is capable of presenting different pieces of information depending on the positions of the watched-side users and where the number of people allows the device to simultaneously present information at different mask levels, such as a lenticular display or stereophonic speakers, the information processing system 1 may perform processing as follows. For example, the information processing system 1 performs presentation to each of the watching-side users at the highest mask level of information set in advance on the basis of the input of the watching users.

Note that, in a case where there is a complete stranger (third party) such as a visitor in the environment of the watching-side users, the information processing system 1 performs presentation at a high mask level of information on the basis of previous setting by the watched-side users. Note that the information processing system 1 may adjust the mask level of information on the basis of an assessment of the situation that is not an explicit instruction based on past information regarding the relationship between the watched-side user and the third party, such as the presence or absence of direct communication with the watched side and the detection frequency.

[1-6-1-3. Examples of Sensing and Data Processing]

Hereinafter, an example of sensing and data processing in the information processing system 1 will be described below.

For example, measurement (sensing) of the state of the watched side may be performed by sensors as the following. Examples of sensors used and acquired information will be described below. The information processing system 1 may perform sensing using a sensor installed in an environment corresponding to the space in which the user is located. For example, the sensor installed in the environment may be a door sensor, a motion sensor, a bed-leaving sensor, an image sensor, a $CO_2$ sensor, a temperature or humidity sensor, an acceleration sensor, a microphone, or the like.

Furthermore, the information processing system 1 may perform sensing using a sensor of a wearable device worn by the user. For example, the sensor of the wearable device may be a sensor that detects the exhalation, swallowing, the pulse, the heartbeat, body motions, or the like or may be a microphone that detects sound or the like.

Furthermore, the information processing system 1 may perform the following data processing. For example, the information processing system 1 may process time-series data acquired from a sensor. The information processing system 1 may perform filtering processing. For example, the filtering processing may be down-sampling, bandpass filtering, outlier removal, clustering, or the like.

The information processing system 1 may perform conversion processing. For example, the information processing system 1 may perform processing based on the presence or absence of a change. For example, the information processing system 1 may perform processing based on a comparison with the latest several weeks or months, a comparison with another day, a comparison with other hours on the same day, or the like.

Furthermore, the information processing system 1 may perform processing based on a difference from another person. For example, the information processing system 1 may perform processing based on a comparison with another person in the same facility or a comparison with a same-age group in another facility. For example, the information processing system 1 may perform dimensionality reduction to an alert to be noted. For example, the information processing system 1 may perform the dimensionality reduction in a case where the activity amount is extremely small or large.

[1-6-1-4. Sensing and Information Presentation Mode]

Note that, in the example of FIG. 1, a case where the abstraction level (mask level) is determined using the input information input by the users has been described as an example. However, the information processing device 100 may determine the abstraction level using any piece of information as long as the information is related to the users. For example, the information processing device 100 may determine the abstraction level (mask level) on the basis of sensing information indicating the state of the user detected by the sensor device 20. For example, the information processing device 100 may determine the abstraction level on the basis of the first sensing information indicating the state of the first user detected by the sensor device 20a. For example, the information processing device 100 may raise the abstraction level in a case where the first user is included in the content that is presentation information. For example, the information processing device 100 may lower the abstraction level in a case where the first user is not included in the content that is presentation information. For example, the information processing device 100 may determine the abstraction level on the basis of the second sensing information indicating the state of the second user detected by the sensor device 20b. For example, in a case where the second user is gazing at the presentation information, the information processing device 100 may lower the abstraction level. For example, in a case where the second user is not gazing at the presentation information, the information processing device 100 may raise the abstraction level.

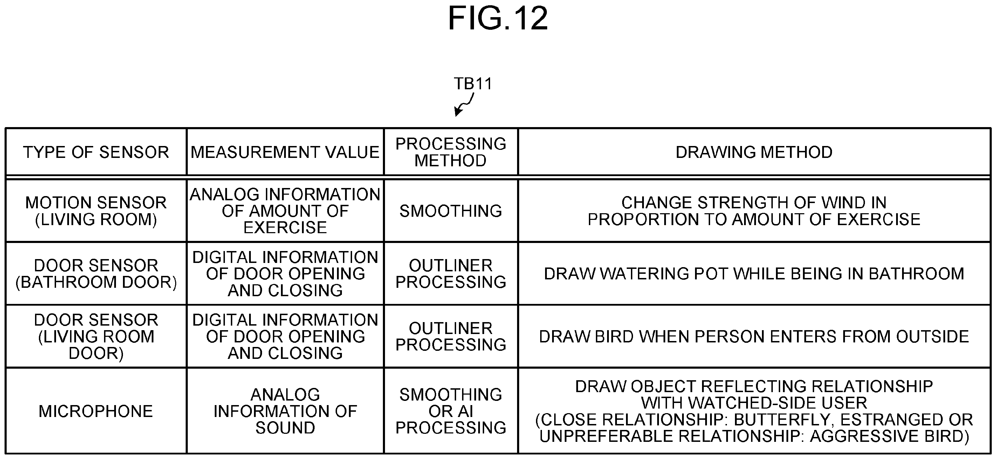

In addition, the information processing system 1 may perform processing or information presentation based on combinations as illustrated in a list TB11 illustrated in FIG. 12. FIG. 12 is a table illustrating examples of sensing targets and presentation modes of information. The list TB11 illustrated in FIG. 12 illustrates mapping of data and presentation methods corresponding to a watched side and a watching side. For example, in a case where the type of the sensor is a motion sensor installed in a living room of the first user, the information processing system 1 performs processing such as smoothing using analog information of the amount of exercise as a measurement value. Then, the information processing system 1 presents the state of the first user by changing the strength of wind in proportion to the amount of exercise.

For example, in a case where the type of the sensor is a door sensor installed in a bathroom door of the first user, the information processing system 1 performs processing such as outlier processing using digital information of door opening or closing as a measurement value. Then, the information processing system 1 presents the state of the first user by drawing a watering pot when the first user is in the bathroom.

For example, in a case where the type of the sensor is a door sensor installed in a living room door of the first user, the information processing system 1 performs processing such as outlier processing using digital information of door opening or closing as a measurement value. Then, the information processing system 1 presents the state of the first user by drawing a bird when a person enters from the outside.

For example, in a case where the type of the sensor is a microphone, the information processing system 1 performs processing such as smoothing or artificial intelligence (AI) processing using analog information of sound as a measurement value. Then, the information processing system 1 presents the state of the first user by drawing an object reflecting the relationship with the watched-side user. For example, the information processing system 1 presents the state of the first user by drawing a person having a close relationship with the first user as a butterfly and a person, who is alienated from the first user or considers the first user unfavorable, as an aggressive bird.

As described above, the information processing system 1 associates an analysis result of data obtained from each sensor with an object to be drawn. In addition, the information processing system 1 draws the degree of deviation of the current situation from the steady state estimated from the past accumulated data in association with the motion of the object or the locus of the motion. As a result, the information processing system 1 can facilitate each user to associate between an event and presentation content even in a case where the mask level of the information is high. For example, the information processing system 1 stores data and the protection level, the attention degree, and the responsiveness in a database in a table format.

For example, the information processing system 1 makes the content matched depending on the intent of the watching side and the watched side. As a result, the information processing system 1 can convey information reflecting the mutually optimum extent of information discloser by the watching side and the watched side. Therefore, the information processing system 1 can implement an interface having high affinity with the daily life through simple operation and elimination of the reality of the presentation content.

Note that, in the above-described example, the sensor information and the presentation content are made to correspond to each other on a one-to-one basis; however, the correspondence relationship is not limited to the one-to-one basis. For example, in a case of an output form in which a user feels uncomfortable when data is missing due to the communication function or a sensor abnormality, such as in a picture, music, or a motion of a robot, the information processing system 1 may calculate an estimation result by sensor fusion using a plurality of other sensors and perform the presentation or may present a latest change. As an example of the sensor fusion, in a case where the value of the door sensor of the living room door repeats 0 and 1 at high speed, the information processing system 1 may determine that the door sensor has an abnormality and estimate the entrance and exit of a person on the basis of data of the microphone and the motion sensor.

[1-6-1-5. Examples of Presentation Device and Presentation Content]

Note that, in the above-described example, the case where the device (presentation device) that presents information is the terminal device 10 such as a smartphone used by the user has been described as an example. However, the presentation device is not limited to the smartphone or the like and may be various devices. That is, the interface (terminal device 10) that presents information in the information processing system 1 is not limited to visual presentation by a two-dimensional video. In addition, the presentation device may not be a dedicated device but an application having an algorithm incorporated into an existing device. In this regard, examples of the presentation device and examples of the presentation content of information in the case of each of the presentation devices will be described below.

For example, the presentation device (for example, the terminal device 10) may be a wearable device such as a smart watch or smart glasses, a three-dimensional (3D) holographic display, or the like. In this case, the presentation content may be a video (type), vibration (type, strength, or periodicity), or others. For example, in the case of a 3D display, the information processing system 1 may present a miniature garden, a three-dimensional avatar video, or the like imitating the room of the watched side.

For example, the presentation device (for example, the terminal device 10) may be a robot or the like. In this case, the presentation content may be the motion (type or strength), a shape change, a rigidity change, a temperature change (type, size, or texture), or others. For example, in the case of a robot, the presentation device may be a small humanoid, a pet-type robot, a flower-shaped robot, an air soft robot, or the like.

For example, the presentation device (for example, the terminal device 10) may be a speaker or the like. In this case, the presentation content may be background music (BGM) (type, volume, tempo, or playlist), a sound effect (type), or others. For example, in the case of a speaker, the information processing system 1 may present up-tempo sound, slow-tempo sound, or others or may present a whistle, breathing sound while sleeping, laughing sound, sighing sound, mastication sound, or the like.

For example, the presentation device (for example, the terminal device 10) may be a diffuser, a heat source, or the like. In this case, the presentation content may be the scent or odor (type or strength), wind (type or strength), the temperature (degree), or the like. For example, in the case of a diffuser, the information processing system 1 may present an aroma of sweet perfume, an aroma of a spicy dish, or others or may present strong sound, weak sound, continuous sound, intermittent sound, or others. For example, in the case of a heat source, the information processing system 1 may present hot or cold.

The information processing system 1 may constantly update the presentation content, update the presentation content only when the user is checking, or may update the presentation content depending on an ON/OFF operation by the user.

In addition, the information processing system 1 may present not only in the real-time scale but also in fast-forwarding in which a long time is compressed to a short time, a highlight in which only events are extracted and connected, a slow loop of an event of a short time, or others. In a case where the environment or the state of the user changes such as the daytime or the nighttime, a shared space or a private room, or the degree of disability, the information processing system 1 may change or adjust the presentation method such as the luminance, the volume, motion, or the like in such a manner that the change can be easily noticed.

Furthermore, the information processing system 1 may detect a place where an observer (watching-side user) is present in cooperation with a plurality of presentation devices to present the place via the closest display. The information processing system 1 may present the degree of steadiness through a comparison not only in a micro environment such as a private room but also in a macro environment such as the entire facility.

As a result, the information processing system 1 can perform communication in a desirable form for the watched side and the watching side.

[1-6-2. System Configuration Example]

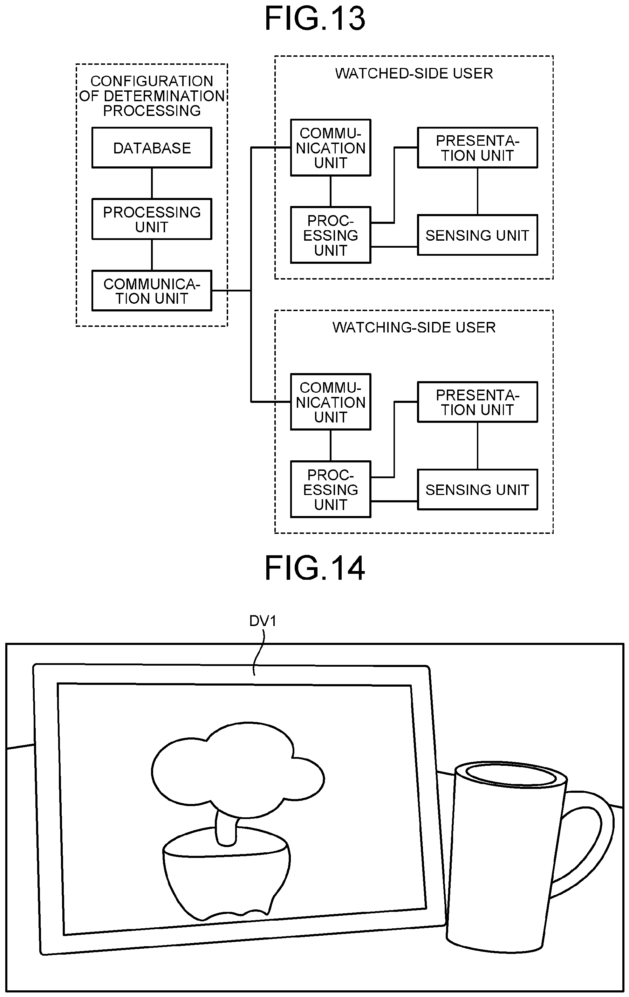

A configuration mode including physical arrangement of components in the information processing system 1 will be conceptually described with reference to FIG. 13. FIG. 13 is a diagram illustrating an example of components of the information processing system.

The information processing system 1 includes a communication unit, a processing unit, a presentation unit, and a sensing unit arranged in correspondence to the watched-side user. For example, the communication unit disposed in correspondence to the watched-side user transmits and receives information to and from components other than the components arranged in correspondence to the watched-side user. The communication unit disposed in correspondence to the watched-side user in FIG. 13 corresponds to the communication unit 11 of the terminal device 10a used by the first user. Furthermore, the processing unit disposed in correspondence to the watched-side user in FIG. 13 corresponds to the control unit 18 of the terminal device 10a used by the first user. Furthermore, the presentation unit disposed in correspondence to the watched-side user in FIG. 13 corresponds to the display unit 15 and the sound output unit 13 of the terminal device 10a used by the first user. The sensing unit disposed corresponding to the watched-side user in FIG. 13 corresponds to the sensor device 20a.

The information processing system 1 includes a communication unit, a processing unit, a presentation unit, and a sensing unit arranged corresponding to the watching-side user. For example, the communication unit disposed in correspondence to the watching-side user transmits and receives information to and from components other than the components arranged in correspondence to the watching-side user. The communication unit disposed in correspondence to the watching-side user in FIG. 13 corresponds to the communication unit 11 of the terminal device 10b used by the second user. Furthermore, the processing unit disposed in correspondence to the watching-side user in FIG. 13 corresponds to the control unit 18 of the terminal device 10b used by the second user. Furthermore, the presentation unit disposed in correspondence to the watching-side user in FIG. 13 corresponds to the display unit 15 and the sound output unit 13 of the terminal device 10b used by the second user. The sensing unit disposed corresponding to the watching-side user in FIG. 13 corresponds to the sensor device 20b.

The information processing system 1 includes a communication unit, a processing unit, and a database arranged in correspondence to the configuration of the determination processing. For example, the communication unit disposed in correspondence to the configuration of the determination processing transmits and receives information to and from components other than the components arranged in correspondence to the configuration of the determination processing. The communication unit disposed in correspondence to the configuration of the determination processing in FIG. 13 corresponds to the communication unit 110 of the information processing device 100. Furthermore, the processing unit disposed in correspondence to the configuration of the determination processing in FIG. 13 corresponds to the control unit 130 of the information processing device 100. Furthermore, the database disposed in correspondence to the configuration of the determination processing in FIG. 13 corresponds to the storage unit 120 of the information processing device 100.

Note that the configuration illustrated in FIG. 13 is a first configuration, and for example, there may be a plurality of watched-side users. Furthermore, there may be a plurality of watching-side users. As described above, as the physical arrangement configuration of the components in the information processing system 1, any configuration can be adopted depending on the presentation information, the presentation mode, or others.

[1-6-3. Examples of Information Presentation]

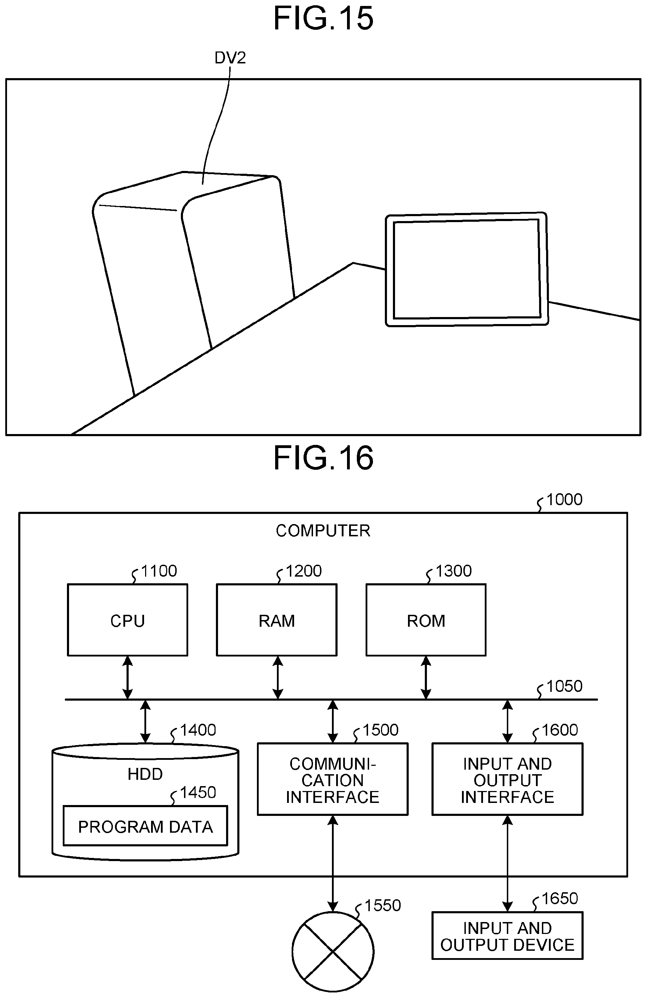

Next, an example of information presentation will be described with reference to FIGS. 14 and 15. FIGS. 14 and 15 are diagrams illustrating an example of information presentation. That is, FIGS. 14 and 15 are diagrams illustrating an example of user interface (UI).

For example, the information processing system 1 may present presentation information by a presentation device DV1 as illustrated in the presentation device DV1 in FIG. 14. In this case, the information processing system 1 may display the presentation information by the presentation device DV1 having a display with a concept in which the degree of blooming of a flower changes. For example, in a case where unsteady sound is detected in a room of the watched side, the information processing system 1 may change the drawing on the watching side.

Note that the information processing system 1 may notify the resident, who is the watched side, of the situation of the family member, who is the watching side. For example, the information processing system 1 may present presentation information by a presentation device DV2 as illustrated in the presentation device DV2 in FIG. 15. In this case, the information processing system 1 may present the presentation information by the presentation device DV2, which is a robot, with a concept in which the behavior of the robot changes. For example, in the information processing system 1, in a case where a grandchild who is the watching side is lively, the presentation device DV2, which is a robot on the watched side, may present information by sound and motion.

[2. Other Embodiments]

The processing according to the above embodiments may be performed in various different modes (modifications) other than in the above embodiments or modifications.

[2-1. Other Configuration Examples]

In the information processing system 1 described above, the terminal devices 10 function as a device that transmits information such as user input and sensing to the information processing device 100 and outputs information received from the information processing device 100, namely, a so-called thin client. As described above, in the above-described example, the information processing system 1 has been described with an exemplary configuration of a so-called centralized system such as a client-server system that executes major processing on the server side. Note that the above is merely an example, and any mode can be adopted for division of functions in the information processing system 1.

For example, in the information processing system 1 of the above-described example, the system configuration in which the information processing device 100 performs the image processing and the speech recognition has been described as an example; however, the image processing and the speech recognition may be performed by each of the terminal devices 10. Furthermore, for example, the terminal devices 10 may perform generation processing or the like of presentation information such as the content. In this case, the terminal devices 10 function as an information processing device that determines the abstraction level and outputs information based on the determined abstraction level. For example, the terminal devices 10 hold information stored in the storage unit 120 and has functions of the processing unit 132, the determination unit 133, and the generation unit 134. For example, the terminal device 10 functions as a rich client that performs processing related to speech recognition and determination, generation, and output (display) of the abstraction level. Furthermore, the information processing device 100 collects information from each of the terminal devices 10 and provides information necessary for each of the terminal devices 10.

Furthermore, for example, in a case where each of the terminal devices 10 communicates or shares information for provision of the watching service between the terminal devices 10 in a so-called peer to peer (P2P) mode, the information processing system 1 is not required to include a server-side device. For example, the information processing system 1 may have a system configuration in which, for example, major processing is executed on the user terminal (client) side, and the server-side device only manages information regarding the watching or in which no server-side device is included, namely, a configuration of a so-called autonomous distributed system. As described above, the information processing system 1 may have either configuration of a centralized configuration or an autonomous distributed configuration.

Note that the configuration described above is an example, and the information processing system 1 may have any function division mode and any device configuration as long as the services related to the watching described above can be provided.

[2-2. Others]

Among the processing described in the above embodiments, the whole or a part of the processing described as that performed automatically can be performed manually, or the whole or a part of the processing described as that performed manually can be performed automatically by a known method. In addition, a processing procedure, a specific name, and information including various types of data or parameters illustrated in the above or in the drawings can be modified as desired unless otherwise specified. For example, various types of information illustrated in the drawings are not limited to the information illustrated.

In addition, each component of each device illustrated in the drawings is conceptual in terms of function and is not necessarily physically configured as illustrated in the drawings. That is, the specific form of distribution or integration of each device is not limited to those illustrated in the drawings, and the whole or a part thereof can be functionally or physically distributed or integrated in any unit depending on various loads, usage status, and others.

In addition, the above embodiments and modifications can be combined as appropriate within a range where there is no conflict in the processing content.

Furthermore, the effects described herein are merely examples and are not limiting, and other effects may be achieved.

[3. Effects of Present Disclosure]

As described above, the information processing device (information processing device 100 in the embodiment) according to the present disclosure includes an acquisition unit (acquisition unit 131 in the embodiment) and a determination unit (determination unit 133 in the embodiment). The acquisition unit acquires the first user information related to the first user who is on the side of being watched over and the second user information related to the second user who is on the side of watching over the first user. The determination unit determines the abstraction level of presentation information that is information indicating the state of the first user on the basis of the first user information and the second user information.

As described above, the information processing device according to the present disclosure can appropriately determine the abstraction level of information by determining the abstraction level of the presentation information that is information indicating the state of the first user on the basis of the first user information related to the first user who is the watched-side user and the second user information related to the second user who is on the side of watching over the first user.

In addition, the acquisition unit acquires first user information including the first input information input by the first user. The determination unit determines the abstraction level of the presentation information of the first user on the basis of the first input information. In this manner, the information processing device can appropriately determine the abstraction level of the information by determining the abstraction level of the presentation information on the basis of the first input information input by the first user.

The acquisition unit also acquires first input information indicating the abstraction level designated by the first user. The determination unit determines the abstraction level of the presentation information of the first user on the basis of the abstraction level indicated by the first input information. In this manner, the information processing device can appropriately determine the abstraction level of the information by determining the abstraction level of the presentation information on the basis of the abstraction level specified by the first user.

In addition, the acquisition unit acquires the first input information indicating an instruction to lower the abstraction level by the first user. The determination unit lowers the abstraction level of the presentation information of the first user in accordance with the instruction to lower the abstraction level by the first user. In this manner, the information processing device can appropriately determine the abstraction level of the information by lowering the abstraction level of the presentation information of the first user in accordance with the instruction to lower the abstraction level by the first user.

In addition, the acquisition unit acquires the first input information indicating an instruction to raise the abstraction level by the first user. The determination unit raises the abstraction level of the presentation information of the first user in accordance with the instruction to raise the abstraction level by the first user. In this manner, the information processing device can appropriately determine the abstraction level of the information by raising the abstraction level of the presentation information of the first user in accordance with the instruction to raise the abstraction level by the first user.

In addition, the acquisition unit acquires the first user information including the first sensing information indicating the state of the first user detected by a sensor. The determination unit determines the abstraction level of the presentation information of the first user on the basis of the first sensing information. In this manner, the information processing device can appropriately determine the abstraction level of the information by determining the abstraction level of the presentation information on the basis of the first sensing information indicating the state of the first user detected by the sensor.

In addition, the acquisition unit acquires second user information including the second input information input by the second user. The determination unit determines the abstraction level of the presentation information of the second user on the basis of the second input information. In this manner, the information processing device can appropriately determine the abstraction level of the information by determining the abstraction level of the presentation information on the basis of the second input information input by the second user.

The acquisition unit also acquires the second input information indicating the abstraction level designated by the second user. The determination unit determines the abstraction level of the presentation information of the second user on the basis of the abstraction level indicated by the second input information. In this manner, the information processing device can appropriately determine the abstraction level of the information by determining the abstraction level of the presentation information on the basis of the abstraction level specified by the second user.

In addition, the acquisition unit acquires the second input information indicating an instruction to lower the abstraction level by the second user. The determination unit lowers the abstraction level of the presentation information of the second user in accordance with the instruction to lower the abstraction level by the second user. In this manner, the information processing device can appropriately determine the abstraction level of the information by lowering the abstraction level of the presentation information of the second user in accordance with the instruction to lower the abstraction level by the second user.

In addition, the acquisition unit acquires the second input information indicating an instruction to raise the abstraction level by the second user. The determination unit raises the abstraction level of the presentation information of the second user in accordance with the instruction to raise the abstraction level by the second user. In this manner, the information processing device can appropriately determine the abstraction level of the information by raising the abstraction level of the presentation information of the second user in accordance with the instruction to raise the abstraction level by the second user.

In addition, the acquisition unit acquires the second user information including the second sensing information indicating the state of the second user detected by a sensor. The determination unit determines the abstraction level of the presentation information of the second user on the basis of the second sensing information. In this manner, the information processing device can appropriately determine the abstraction level of the information by determining the abstraction level of the presentation information on the basis of the second sensing information indicating the state of the second user detected by the sensor.

In addition, the determination unit determines the abstraction level at the time of presenting the presentation information of the first user to the second user on the basis of the first user information and the second user information. In this manner, the information processing device can appropriately determine the abstraction level of the information by determining the abstraction level at the time of presenting the presentation information to the second user.

In addition, the determination unit determines the abstraction level at the time of presenting the presentation information of the first user to the first user on the basis of the first user information and the second user information. In this manner, the information processing device can appropriately determine the abstraction level of the information by determining the abstraction level at the time of presenting the presentation information to the first user.

In addition, the determination unit determines the abstraction level of the presentation information of the first user depending on the protection level of the presentation information of the first user determined on the basis of the first user information and the second user information. In this manner, the information processing device can appropriately determine the abstraction level of the information by determining the abstraction level of the presentation information of the first user depending on the protection level of the presentation information determined on the basis of the first user information and the second user information.

Furthermore, the determination unit determines the amount of information of the presentation information of the first user depending on the attention degree of the presentation information of the first user determined on the basis of the first user information and the second user information. In this manner, the information processing device can appropriately determine the amount of information by determining the amount of information of the presentation information of the first user depending on the attention degree of the presentation information determined on the basis of the first user information and the second user information.

Furthermore, the determination unit determines the resolution of the presentation information of the first user depending on the responsiveness of the presentation information of the first user determined on the basis of the first user information and the second user information. In this manner, the information processing device can appropriately determine the resolution of the information by determining the resolution of the presentation information of the first user depending on the responsiveness of the presentation information determined on the basis of the first user information and the second user information.

Furthermore, the information processing device includes an output unit (the transmission unit 135 in the embodiment). The output unit outputs the presentation information corresponding to the abstraction level determined by the determination unit. In this manner, the information processing device can output information of an appropriate abstraction level by outputting the presentation information corresponding to the determined abstraction level.

Furthermore, the output unit transmits the presentation information corresponding to the abstraction level determined by the determination unit to the terminal device used by the second user. In this manner, the information processing device can provide information of an appropriate abstraction level by transmitting the presentation information corresponding to the abstraction level to the terminal device used by the second user.

Furthermore, the output unit transmits the presentation information corresponding to the abstraction level determined by the determination unit to the terminal device used by the first user. In this manner, the information processing device can provide information of an appropriate abstraction level by transmitting the presentation information corresponding to the abstraction level to the terminal device used by the first user.

[4. Hardware Configuration]

Information processing devices (information appliances) such as the information processing device 100 and the terminal devices 10 according to the embodiments described above are implemented by, for example, a computer 1000 having a configuration as illustrated in FIG. 16. FIG. 16 is a hardware configuration diagram illustrating an example of the computer 1000 that implements the functions of the information processing device. Hereinafter, the information processing device 100 according to an embodiment will be described as an example. The computer 1000 includes a CPU 1100, a RAM 1200, a read only memory (ROM) 1300, a hard disk drive (HDD) 1400, a communication interface 1500, and an input and output interface 1600. The components of the computer 1000 are connected by a bus 1050.

The CPU 1100 operates in accordance with a program stored in the ROM 1300 or the HDD 1400 and controls each of the components. For example, the CPU 1100 loads a program stored in the ROM 1300 or the HDD 1400 in the RAM 1200 and executes processing corresponding to various programs.

The ROM 1300 stores a boot program such as a basic input output system (BIOS) executed by the CPU 1100 when the computer 1000 is activated, a program dependent on the hardware of the computer 1000, and the like.

The HDD 1400 is a computer-readable recording medium that non-transiently records a program to be executed by the CPU 1100, data used by such a program, and the like. Specifically, the HDD 1400 is a recording medium that records an information processing program according to the present disclosure, which is an example of program data 1450.

The communication interface 1500 is an interface for the computer 1000 to be connected with an external network 1550 (for example, the Internet). For example, the CPU 1100 receives data from another device or transmits data generated by the CPU 1100 to another device via the communication interface 1500.

The input and output interface 1600 is an interface for connecting an input and output device 1650 and the computer 1000. For example, the CPU 1100 receives data from an input device such as a keyboard or a mouse via the input and output interface 1600. The CPU 1100 also transmits data to an output device such as a display, a speaker, or a printer via the input and output interface 1600. Furthermore, the input and output interface 1600 may function as a media interface that reads a program or the like recorded in a predetermined recording medium. A medium refers to, for example, an optical recording medium such as a digital versatile disc (DVD) or a phase change rewritable disk (PD), a magneto-optical recording medium such as a magneto-optical disk (MO), a tape medium, a magnetic recording medium, or a semiconductor memory.

For example, in a case where the computer 1000 functions as the information processing device 100 according to the embodiment, the CPU 1100 of the computer 1000 implements the functions of the control unit 130 or other units by executing the information processing program loaded on the RAM 1200. The HDD 1400 also stores the information processing program according to the present disclosure or data in the storage unit 120. Note that although the CPU 1100 reads the program data 1450 from the HDD 1400 and executes the program data 1450, as another example, these programs may be acquired from another device via the external network 1550.

Note that the present technology can also have the following configurations.

(1) An information processing device comprising:

an acquisition unit that acquires first user information related to a first user who is a user on a side to be watched over and second user information related to a second user who is a user on a side of watching over the first user; and
a determination unit that determines an abstraction level of presentation information that is information indicating a state of the first user on a basis of the first user information and the second user information.

(2) The information processing device according to (1), wherein

the acquisition unit
acquires the first user information including first input information input by the first user, and
the determination unit
determines the abstraction level of the presentation information of the first user on a basis of the first input information.

(3) The information processing device according to (2), wherein

the acquisition unit
acquires the first input information indicating an abstraction level designated by the first user, and
the determination unit
determines the abstraction level of the presentation information of the first user on a basis of the abstraction level indicated by the first input information.

(4) The information processing device according to (3), wherein

the acquisition unit
acquires the first input information indicating an instruction to lower an abstraction level by the first user, and
the determination unit
lowers the abstraction level of the presentation information of the first user in accordance with the instruction to lower the abstraction level by the first user.

(5) The information processing device according to (3) or (4), wherein

the acquisition unit
acquires the first input information indicating an instruction to raise an abstraction level by the first user, and
the determination unit

raises the abstraction level of the presentation information of the first user in accordance with the instruction to raise the abstraction level by the first user.

(6) The information processing device according to any one of (1) to (5), wherein

the acquisition unit
acquires the first user information including first sensing information indicating a state of the first user detected by a sensor, and
the determination unit
determines the abstraction level of the presentation information of the first user on a basis of the first sensing information.

(7) The information processing device according to any one of (1) to (6), wherein

the acquisition unit
acquires the second user information including second input information input by the second user, and
the determination unit
determines an abstraction level of the presentation information of the second user on a basis of the second input information.

(8) The information processing device according to (7), wherein

the acquisition unit
acquires the second input information indicating an abstraction level designated by the second user, and
the determination unit
determines the abstraction level of the presentation information of the second user on a basis of the abstraction level indicated by the second input information.

(9) The information processing device according to (8), wherein

the acquisition unit
acquires the second input information indicating an instruction to lower an abstraction level by the second user, and
the determination unit
lowers the abstraction level of the presentation information of the second user in accordance with the instruction to lower the abstraction level by the second user.

(10) The information processing device according to (8) or (9), wherein

the acquisition unit
acquires the second input information indicating an instruction to raise an abstraction level by the second user, and
the determination unit
raises the abstraction level of the presentation information of the second user in accordance with the instruction to raise the abstraction level by the second user.

(11) The information processing device according to any one of (1) to (10), wherein

the acquisition unit
acquires the second user information including second sensing information indicating a state of the second user detected by a sensor, and
the determination unit
determines an abstraction level of the presentation information of the second user on a basis of the second sensing information.

(12) The information processing device according to any one of (1) to (11), wherein

the determination unit

determines an abstraction level at a time of presenting the presentation information of the first user to the second user on a basis of the first user information and the second user information.

(13) The information processing device according to any one of (1) to (12), wherein

the determination unit
determines an abstraction level at a time of presenting the presentation information of the first user to the first user on a basis of the first user information and the second user information.

(14) The information processing device according to any one of (1) to (13), wherein

the determination unit
determines the abstraction level of the presentation information of the first user depending on a protection level of the presentation information of the first user determined on a basis of the first user information and the second user information.

(15) The information processing device according to any one of (1) to (14), wherein

the determination unit
determines an amount of information of the presentation information of the first user depending on an attention degree of the presentation information of the first user determined on a basis of the first user information and the second user information.

(16) The information processing device according to any one of (1) to (15), wherein

the determination unit
determines a resolution of the presentation information of the first user depending on responsiveness of the presentation information of the first user determined on a basis of the first user information and the second user information.

(17) The information processing device according to any one of (1) to (16), further comprising:
an output unit that outputs the presentation information corresponding to the abstraction level determined by the determination unit.
(18) The information processing device according to (17), wherein

the output unit
transmits the presentation information corresponding to the abstraction level determined by the determination unit to a terminal device used by the second user.

(19) The information processing device according to (17) or (18), wherein

the output unit
transmits the presentation information corresponding to the abstraction level determined by the determination unit to a terminal device used by the first user.

(20) An information processing method comprising:

acquiring first user information related to a first user who is a user on a side to be watched over and second user information related to a second user who is a user on a side of watching over the first user; and
determining an abstraction level of presentation information that is information indicating a state of the first user on a basis of the first user information and the second user information.

Reference Signs List

1    INFORMATION PROCESSING SYSTEM

100    INFORMATION PROCESSING DEVICE
110    COMMUNICATION UNIT
120    STORAGE UNIT
121    DATA STORAGE UNIT
122    USER INFORMATION STORAGE UNIT
130    CONTROL UNIT
131    ACQUISITION UNIT
132    PROCESSING UNIT
133    DETERMINATION UNIT
134    GENERATION UNIT
135    TRANSMISSION UNIT
10     TERMINAL DEVICE (OUTPUT DEVICE)
11     COMMUNICATION UNIT
12     VOICE INPUT UNIT (INPUT UNIT)
13     SOUND OUTPUT UNIT
14     CAMERA
15     DISPLAY UNIT
16     OPERATION UNIT (INPUT UNIT)
17     STORAGE UNIT
18     CONTROL UNIT
181    ACQUISITION UNIT
182    TRANSMISSION UNIT
183    RECEPTION UNIT
184    PROCESSING UNIT
20     SENSOR DEVICE

**Claims**

1.  An information processing device comprising:

    an acquisition unit that acquires first user information related to a first user who is a user on a side to be watched over and second user information related to a second user who is a user on a side of watching over the first user; and
    a determination unit that determines an abstraction level of presentation information that is information indicating a state of the first user on a basis of the first user information and the second user information.

2.  The information processing device according to claim 1, wherein

    the acquisition unit
    acquires the first user information including first input information input by the first user, and
    the determination unit
    determines the abstraction level of the presentation information of the first user on a basis of the first input information.

3.  The information processing device according to claim 2, wherein

    the acquisition unit
    acquires the first input information indicating an abstraction level designated by the first user, and
    the determination unit
    determines the abstraction level of the presentation information of the first user on a basis of the abstraction level indicated by the first input information.

4.  The information processing device according to claim 3, wherein

    the acquisition unit
    acquires the first input information indicating an instruction to lower an abstraction level by the first user, and
    the determination unit
    lowers the abstraction level of the presentation information of the first user in accordance with the instruction to lower the abstraction level by the first user.

5. The information processing device according to claim 3, wherein

   the acquisition unit
   acquires the first input information indicating an instruction to raise an abstraction level by the first user, and
   the determination unit
   raises the abstraction level of the presentation information of the first user in accordance with the instruction to raise the abstraction level by the first user.

6. The information processing device according to claim 1, wherein

   the acquisition unit
   acquires the first user information including first sensing information indicating a state of the first user detected by a sensor, and
   the determination unit
   determines the abstraction level of the presentation information of the first user on a basis of the first sensing information.

7. The information processing device according to claim 1, wherein

   the acquisition unit
   acquires the second user information including second input information input by the second user, and
   the determination unit
   determines an abstraction level of the presentation information of the second user on a basis of the second input information.

8. The information processing device according to claim 7, wherein

   the acquisition unit
   acquires the second input information indicating an abstraction level designated by the second user, and
   the determination unit
   determines the abstraction level of the presentation information of the second user on a basis of the abstraction level indicated by the second input information.

9. The information processing device according to claim 8, wherein

   the acquisition unit
   acquires the second input information indicating an instruction to lower an abstraction level by the second user, and
   the determination unit
   lowers the abstraction level of the presentation information of the second user in accordance with the instruction to lower the abstraction level by the second user.

10. The information processing device according to claim 8, wherein

    the acquisition unit
    acquires the second input information indicating an instruction to raise an abstraction level by the second user, and
    the determination unit
    raises the abstraction level of the presentation information of the second user in accordance with the instruction to raise the abstraction level by the second user.

11. The information processing device according to claim 1, wherein

    the acquisition unit
    acquires the second user information including second sensing information indicating a state of the second user detected by a sensor, and

the determination unit

determines an abstraction level of the presentation information of the second user on a basis of the second sensing information.

12. The information processing device according to claim 1, wherein

the determination unit

determines an abstraction level at a time of presenting the presentation information of the first user to the second user on a basis of the first user information and the second user information.

13. The information processing device according to claim 1, wherein

the determination unit

determines an abstraction level at a time of presenting the presentation information of the first user to the first user on a basis of the first user information and the second user information.

14. The information processing device according to claim 1, wherein

the determination unit

determines the abstraction level of the presentation information of the first user depending on a protection level of the presentation information of the first user determined on a basis of the first user information and the second user information.

15. The information processing device according to claim 1, wherein

the determination unit

determines an amount of information of the presentation information of the first user depending on an attention degree of the presentation information of the first user determined on a basis of the first user information and the second user information.

16. The information processing device according to claim 1, wherein

the determination unit

determines a resolution of the presentation information of the first user depending on responsiveness of the presentation information of the first user determined on a basis of the first user information and the second user information.

17. The information processing device according to claim 1, further comprising:
an output unit that outputs the presentation information corresponding to the abstraction level determined by the determination unit.

18. The information processing device according to claim 17, wherein

the output unit

transmits the presentation information corresponding to the abstraction level determined by the determination unit to a terminal device used by the second user.

19. The information processing device according to claim 17, wherein

the output unit

transmits the presentation information corresponding to the abstraction level determined by the determination unit to a terminal device used by the first user.

20. An information processing method comprising:

acquiring first user information related to a first user who is a user on a side to be watched over and second user information related to a second user who is a user on a side of watching over the first user; and
determining an abstraction level of presentation information that is information indicating a state of the first user on a basis of the first user information and the second user information.

# FIG.1

# FIG.2

MASK LEVEL OF
INFORMATION

GR1

INITIAL VALUE OF
WATCHING SIDE
$ML_{initial\_elder}$

LN1

LN2

INITIAL VALUE OF
WATCHED SIDE
$ML_{initial\_younger}$

t0

TIME

# FIG.3

MASK LEVEL FOR PRESENTATION AT TIME t:$ML(t)$
INITIAL VALUE OF MASK LEVEL SET BY WATCHED SIDE:$ML_{initial\_elder}$
FEEDBACK AT TIME t FROM WATCHED SIDE:$FB(t)_{elder}$
WEIGHTING COEFFICIENT OF FEEDBACK FROM WATCHED SIDE: $\alpha(t)_{elder}$
INITIAL VALUE OF MASK LEVEL SET BY WATCHING SIDE:$ML_{initial\_younger}$
FEEDBACK AT TIME t FROM WATCHING SIDE:$FB(t)_{younger}$
WEIGHTING COEFFICIENT OF FEEDBACK FROM WATCHING SIDE: $\beta(t)_{younger}$

# FIG.4

PS1

HIGH                    PROTECTION LEVEL                    LOW

EP 4 503 595 A1

# FIG.5

INFORMATION PROCESSING DEVICE ⌐100

1

(WATCHED SIDE)

⌐10a(10)
TERMINAL DEVICE

⌐20a(20)
SENSOR DEVICE

N

(WATCHING SIDE)

⌐10b(10)
TERMINAL DEVICE

⌐20b(20)
SENSOR DEVICE

# FIG.6

⌐100

INFORMATION PROCESSING DEVICE

⌐110
COMMUNICATION UNIT

⌐130
CONTROL UNIT

⌐131
ACQUISITION UNIT

⌐132
PROCESSING UNIT

⌐133
DETERMINATION UNIT

⌐134
GENERATION UNIT

⌐135
TRANSMISSION UNIT

⌐120
STORAGE UNIT

⌐121
DATA STORAGE UNIT

⌐122
USER INFORMATION STORAGE UNIT

⌐123
ABSTRACTION LEVEL INFORMATION STORAGE UNIT

# FIG.7

TERMINAL DEVICE — 10

COMMUNICATION UNIT — 11

CONTROL UNIT — 18

CAMERA — 14

ACQUISITION UNIT — 181

TRANSMISSION UNIT — 182

DISPLAY UNIT — 15

VOICE INPUT UNIT — 12

RECEPTION UNIT — 183

OPERATION UNIT — 16

PROCESSING UNIT — 184

SOUND OUTPUT UNIT — 13

STORAGE UNIT — 17

# FIG.8

START

ACQUIRE FIRST USER INFORMATION RELATED TO FIRST USER WHO IS ON WATCHED SIDE AND SECOND USER INFORMATION RELATED TO SECOND USER WHO IS ON SIDE OF WATCHING OVER FIRST USER — S101

DETERMINE ABSTRACTION LEVEL OF PRESENTATION INFORMATION THAT IS INFORMATION INDICATING STATE OF FIRST USER ON BASIS OF FIRST USER INFORMATION AND SECOND USER INFORMATION — S102

END

# FIG.9

START

S201
REFER TO DB

S202
SENSING ON WATCHED SIDE

S203
FILTERING, CONVERSION,
AND STATE ESTIMATION

S204
IS THERE INPUT ON
WATCHED SIDE?　　YES

NO

S205
UPDATE PRESENTATION CONTENT

S206
IS THERE INPUT ON
WATCHING SIDE?　　YES

NO

S207
UPDATE PRESENTATION CONTENT

S208
UPDATE DB

# FIG.10

MASK LEVEL OF
INFORMATION

GR2

INITIAL VALUE OF
WATCHING SIDE
$ML_{initial\_elder}$

INITIAL VALUE OF
WATCHED SIDE
$ML_{initial\_younger}$

LN11
LN12
LN22
LN21

TIME

t0

# FIG.11

MASK LEVEL OF PRESENTATION TO WATCHED SIDE AT TIME t:$ML_{elder}(t)$
INITIAL VALUE OF MASK LEVEL SET BY WATCHED SIDE:$ML_{initial\_elder}$
FEEDBACK AT TIME t FROM WATCHED SIDE:$FB(t)_{elder}$
WEIGHTING COEFFICIENT OF FEEDBACK FROM WATCHED SIDE: $\alpha(t)_{elder}$
MASK LEVEL OF PRESENTATION TO WATCHING SIDE AT TIME t:$ML_{younger}(t)$
INITIAL VALUE OF MASK LEVEL SET BY WATCHING SIDE:$ML_{initial\_younger}$
FEEDBACK AT TIME t FROM WATCHING SIDE:$FB(t)_{younger}$
WEIGHTING COEFFICIENT OF FEEDBACK FROM WATCHING SIDE: $\beta(t)_{younger}$

## FIG.12

TB11

| TYPE OF SENSOR | MEASUREMENT VALUE | PROCESSING METHOD | DRAWING METHOD |
|---|---|---|---|
| MOTION SENSOR (LIVING ROOM) | ANALOG INFORMATION OF AMOUNT OF EXERCISE | SMOOTHING | CHANGE STRENGTH OF WIND IN PROPORTION TO AMOUNT OF EXERCISE |
| DOOR SENSOR (BATHROOM DOOR) | DIGITAL INFORMATION OF DOOR OPENING AND CLOSING | OUTLINER PROCESSING | DRAW WATERING POT WHILE BEING IN BATHROOM |
| DOOR SENSOR (LIVING ROOM DOOR) | DIGITAL INFORMATION OF DOOR OPENING AND CLOSING | OUTLINER PROCESSING | DRAW BIRD WHEN PERSON ENTERS FROM OUTSIDE |
| MICROPHONE | ANALOG INFORMATION OF SOUND | SMOOTHING OR AI PROCESSING | DRAW OBJECT REFLECTING RELATIONSHIP WITH WATCHED-SIDE USER (CLOSE RELATIONSHIP: BUTTERFLY, ESTRANGED OR UNPREFERABLE RELATIONSHIP: AGGRESSIVE BIRD) |

# FIG.13

```
┌─────────────────────────┐   ┌──────────────────────────────────────────┐
│ CONFIGURATION           │   │        WATCHED-SIDE USER                   │
│ OF DETERMINATION        │   │                                            │
│ PROCESSING              │   │   ┌──────────┐        ┌──────────────┐      │
│                         │   │   │ COMMU-   │        │ PRESENTA-    │      │
│  ┌──────────────────┐   │   │   │ NICATION │        │ TION UNIT    │      │
│  │    DATABASE      │   │   │   │ UNIT     │        │              │      │
│  └──────────────────┘   │   │   └──────────┘        └──────────────┘      │
│          │              │   │                                            │
│  ┌──────────────────┐   │   │   ┌──────────┐        ┌──────────────┐      │
│  │   PROCESSING     │   │   │   │ PROC-    │        │ SENSING UNIT │      │
│  │     UNIT         │   │   │   │ ESSING   │        │              │      │
│  └──────────────────┘   │   │   │ UNIT     │        └──────────────┘      │
│          │              │   │   └──────────┘                             │
│  ┌──────────────────┐   │   └──────────────────────────────────────────┘
│  │  COMMUNICA-      │   │
│  │  TION UNIT       │   │   ┌──────────────────────────────────────────┐
│  └──────────────────┘   │   │        WATCHING-SIDE USER                  │
└─────────────────────────┘   │                                            │
                              │   ┌──────────┐        ┌──────────────┐      │
                              │   │ COMMU-   │        │ PRESENTA-    │      │
                              │   │ NICATION │        │ TION UNIT    │      │
                              │   │ UNIT     │        └──────────────┘      │
                              │   └──────────┘                             │
                              │   ┌──────────┐        ┌──────────────┐      │
                              │   │ PROC-    │        │ SENSING UNIT │      │
                              │   │ ESSING   │        │              │      │
                              │   │ UNIT     │        └──────────────┘      │
                              │   └──────────┘                             │
                              └──────────────────────────────────────────┘
```

# FIG.14

# FIG.15

DV2

# FIG.16

1000

COMPUTER

1100
CPU

1200
RAM

1300
ROM

1050

1400
HDD
1450
PROGRAM DATA

1500
COMMUNI-
CATION
INTERFACE

1600
INPUT AND
OUTPUT
INTERFACE

1550

1650
INPUT AND
OUTPUT DEVICE

## INTERNATIONAL SEARCH REPORT

International application No.

**PCT/JP2023/010453**

### A. CLASSIFICATION OF SUBJECT MATTER

*H04N 7/14*(2006.01)i; *G06F 3/0481*(2022.01)i; *G06F 3/0484*(2022.01)i; *G06F 3/16*(2006.01)i; *G06T 13/20*(2011.01)i; *G16H 50/00*(2018.01)i; *H04L 67/565*(2022.01)i; *H04M 11/00*(2006.01)i
FI:   H04N7/14 110; G06F3/16 650; G06F3/0484; G06F3/0481; G06T13/20; G16H50/00; H04L67/565; H04M11/00 301

According to International Patent Classification (IPC) or to both national classification and IPC

### B. FIELDS SEARCHED

Minimum documentation searched (classification system followed by classification symbols)

H04N7/14; G06F3/0481; G06F3/0484; G06F3/16; G06T13/20; G16H50/00; H04L67/565; H04M11/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2005-078138 A (TOSHIBA CORP.) 24 March 2005 (2005-03-24) paragraphs [0017], [0019], [0030], [0047]-[0054], fig. 1, 6, 8 | 1-20 |
| A | JP 2017-117492 A (HITACHI, LTD.) 29 June 2017 (2017-06-29) entire text, all drawings | 1-20 |
| A | JP 2016-154320 A (KDDI CORP.) 25 August 2016 (2016-08-25) entire text, all drawings | 1-20 |
| A | JP 2010-122914 A (SECOM CO., LTD.) 03 June 2010 (2010-06-03) entire text, all drawings | 1-20 |

☐ Further documents are listed in the continuation of Box C.   ☑ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **16 May 2023** | **30 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

<div align="center">

**INTERNATIONAL SEARCH REPORT**
Information on patent family members

</div>

| International application No. |
| --- |
| **PCT/JP2023/010453** |

| Patent document cited in search report | | | Publication date (day/month/year) | Patent family member(s) | Publication date (day/month/year) |
| --- | --- | --- | --- | --- | --- |
| JP | 2005-078138 | A | 24 March 2005 | (Family: none) | |
| JP | 2017-117492 | A | 29 June 2017 | (Family: none) | |
| JP | 2016-154320 | A | 25 August 2016 | (Family: none) | |
| JP | 2010-122914 | A | 03 June 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006285308 A